# EUROPEAN PATENT APPLICATION

(11) **EP 4 385 513 A1**
(43) Date of publication of application: **19.06.2024**
(21) Application number: 22306913.9
(22) Date of filing: 16.12.2022
(51) Int. Cl.: A61K 31/609, A61K 31/198, A61K 31/5377, A61P 5/06

(54) **HALOGENATED SALICYLANILIDE DERIVATIVES AS USP8 INHIBITORS FOR THERAPEUTIC USES**

(71) Applicant: Commissariat à l'Energie Atomique et aux Energies Alternatives, 75015 Paris (FR); INSTITUT NATIONAL DE LA SANTE ET DE LA RECHERCHE MEDICALE - INSERM, 75013 Paris (FR); Université Grenoble Alpes, 38400 Saint-Martin-d'Hères (FR)
(72) Inventor: FAUVARQUE, Marie-Odile, 38054 GRENOBLE CEDEX 09 (FR); WALTRICH AUGUSTO, Thierry, 38054 GRENOBLE CEDEX 09 (FR); MORTIER, Magda, 38054 GRENOBLE CEDEX 09 (FR); BARETTE, Caroline, 38054 GRENOBLE CEDEX 09 (FR)
(74) Representative: Cabinet Nony

(57) **Abstract**

The invention relates to compounds of general formula (I): 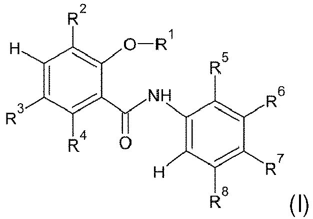 for use for treating an adrenocorticotropin hormone (ACTH)-dependent Cushing's syndrome, wherein R¹ is H or a -C(O)-R⁹, wherein R⁹ is a C₁-C₃ linear or branched, saturated or unsaturated, alkyl group; R² is H or a halogen; R³ is a halogen; R⁴ and R⁸ are, independently, H or a halogen, R⁵ is H, a C₁-C₃, linear or branched, saturated or unsaturated, alkyl group, OH, or a halogen; R⁶ is H, a halogen, or -C(O)-Ar, wherein Ar is a C₆-C₁₀ aryl group, Ar being optionally substituted with a C₁-C₃ linear or branched, saturated or unsaturated, alkyl group; R⁷ is H, a halogen, a C₁-C₃, linear or branched, saturated or unsaturated, alkyl group, -NO₂, or -B-Ar, wherein B is O or -C(CN)-, and Ar is a C₆-C₁₀ aryl group, Ar being optionally substituted with a halogen, wherein if R² is H, then at least one of R⁵, R⁶, R⁷ and R⁸ is not H,or one of its pharmaceutically acceptable salts.

## Description

### [FIELD]

The present invention concerns halogenated salicylanilides derivatives for use in the treatment of an adrenocorticotropin hormone (ACTH)-dependent Cushing's syndrome. The invention also relates to a method for identifying Ubiquitin Specific Protease (USP), or other ubiquitin proteases of the deubiquitinylating enzymes family, inhibitors, in particular reversible inhibitors.

### [BACKGROUND]

First-line treatment of Cushing's disease is trans-sphenoidal surgery allowing a remission rate of 70 to 80% (Cristante et al., 2019; Lefournier et al., 2003; Pivonello et al., 2015; Sarkis et al., 2018). However, other therapeutic strategies are mandatory for the patients who are initially not cured by neurosurgery and or who show recurrences after neurosurgery. These strategies include radiotherapy, bilateral adrenalectomy (and lifelong glucocorticoid replacement therapy) or the use of drugs that inhibit either pituitary ACTH release or adrenal cortisol synthesis or block activation of the glucocorticoid receptor. These drugs all suffer from a limited efficacy and produce many side effects, some of which severe. As for examples, Metyrapone inhibits cortisol synthesis and the antimycotic drug ketoconazole inhibits the early steps of steroidogenesis. Ketoconazole, however, also inhibits androgen synthesis and may cause liver damage (Lonser et al., 2017; Pivonello et al., 2015; Sarkis et al., 2018).

In pituitary corticotroph cells, the Epidermal Growth Factor Receptor (EGFR) may activate a Mitogen Activated Protein Kinase (MAPK)-dependent pathway that could stimulate the expression of the ACTH precursor proopiomelanocortin (POMC) and ACTH secretion (Perez-Rivas and Reincke, 2016). Accordingly, inhibitors of EGFR, such as Gefitinib or Lapatinib, down-regulate POMC expression and ACTH secretion in cultured pituitary cells and have been proposed as therapeutic drugs for suppressing ACTH in corticotroph adenomas (Asari et al., 2019; Ben-Shlomo and Cooper, 2017; Ma et al., 2015).

The EGFR is one of the main targets of USP8 which degradation and recycling is tightly controlled by ubiquitination.

Three independent human genetic sequencing projects have revealed that the gene encoding the ubiquitin specific protease 8 (USP8) is frequently mutated in tumors from Cushing's disease patients (Ma et al., 2015; Perez-Rivas et al., 2018; Reincke et al., 2015). USP8 (also known as UBPY) is a cysteine protease that belongs to the ubiquitin-specific processing protease family. The USP8 is a protein with deubiquitinase (DUB) activity that inhibits the lysosomal degradation of epidermal growth factor receptor (EGFR).

Mutations in USP8, identified in the context of Cushing's disease, all cluster in a region upstream of the catalytic domain that binds the 14-3-3 adaptor protein (LKRSYS718SP720). The 14-3-3 protein functions as a negative regulator of USP8 activity. The most frequent mutation on USP8 affects the serine residue S718, the phosphorylation of which is necessary for 14-3-3 binding, and/or the close proline P720, that is also a key residue for 14-3-3 binding. These mutations are dominant: they abolish or reduce the ability of USP8 to bind 14-3-3, causing a constitutive deubiquitinating activity and subsequent deregulation of USP8 targets and cell homeostasis. Interestingly, no such USP8 mutations were detected in non-corticotroph pituitary adenomas, indicating a high specificity of ACTH-secreting adenomas.

Gain-of-function mutations of USP8 found in Cushing's disease patients interfere with EGFR trafficking to lysosomes and degradation and favor its recycling back to the plasma membrane (Reincke et al., 2015). Stabilization of EGFR in cells expressing USP8 gain-of-function mutations may therefore be the main cause of enhanced POMC expression and ACTH production.

USP8 is thus a promising target in a therapeutic perspective for Cushing's disease patients. This is particularly the case for patients carrying USP8 gain-of-function mutations in view of personalized treatment, and possibly for patients with other alterations if USP8 inhibition allows efficient decrease in ACTH secretion per se. Presently, published or patented USP8 enzymatic inhibitors require further development (Fauvarque et al., 2016; Ritorto et al., 2014; Colombo et al., 2010; Han et al., 2020; Tian et al., 2022; Treppiedi et al., 2021).

Noteworthy, USP8 gene silencing in primary USP8-mutated corticotroph adenoma cells efficiently reduced ACTH secretion (Fauvarque et al., 2016; Han et al., 2020; Jian et al., 2016; Tian et al., 2022; Treppiedi et al., 2021).

Cell treatment with previously described USP8 inhibitors reduce POMC expression levels and ACTH secretion in AtT-20 mice pituitary cells (Han et al., 2020; Jian et al., 2015; Treppiedi et al., 2021). These results indicate that inhibiting USP8 is a relevant strategy to reduce ACTH secretion.

Existing inhibitors of USP8 are insufficiently selective and/or present excessive toxicity and/or cause irreversible oxidation of the catalytic site of ubiquitin protease and/or may react with endogenous proteins and/or are not yet used in clinic.

Therefore, there is a need for additional compounds able to selectively inhibit USP enzymes, such as USP8, relative to other proteases.

There is a need for compounds able to inhibit USP8 with low or now toxicity.

There is a need for reversible inhibitor of USP. Further, there is need for reversible USP8 inhibitors.

There is a need for compounds able to inhibit USP without causing irreversible oxidation of the catalytic site of ubiquitin protease. Further, there is a need for compounds able to inhibit USP8 without causing irreversible oxidation of the catalytic site of ubiquitin protease

There is a need for USP inhibitors with low or no toxicity. Further, there is a need for USP8 inhibitors with low or no toxicity.

There is a need for USP inhibitors with high selectivity and specificity. Further, there is a need for USP8 inhibitors with high selectivity and specificity.

There is a need for compounds able to inhibit USP enzymes, such as USP8, with no or low cross-reaction with other endogenous proteases.

There is a need for such compounds for treating an ACTH-dependent Cushing's syndrome, including Cushing's disease.

Deubiquitinating enzymes (DUB) activity relies on a conserved catalytic cysteine residue which is particularly sensitive to oxidation by reactive oxygen species (ROS) (Lee et al., 2013). Thus, redox cycling compounds can generate µM concentrations of hydrogen peroxide (H₂O₂) in buffer containing strong reducing reagents such as dithiothreitol (DTT). H₂O₂ generated by such redox cycling molecules can then indirectly inhibit the catalytic activity of proteins by oxidizing accessible cysteine, tryptophan, methionine, histidine or selenocysteine residues. Oxidation of USP2 has been notably described to occur in the presence of both quinone containing compounds and DTT (Gopinath et al., 2016; Johnston, 2011).

Previously described naphtoquinones inhibiting the enzymatic activity of USP8 displayed potent and relatively selective inhibition of USP8 compared to other USPs

(Fauvarque et al., 2016). However, the quinone may act as an oxidizing group potentially inducing lack of selectivity and cell toxicity (Gopinath et al., 2016; Johnston, 2011).

Therefore, there is a need for a method for identifying new and selective USP inhibitors, such as USP8 inhibitors, not acting through oxidation of the catalytic cysteine.

There is a need for a method for identifying reversible USP inhibitors, such as reversible USP8 inhibitors.

The present disclosure has for purpose to satisfy all or part of those needs.

### [SUMMARY]

The present disclosure relates halogenated salicylanilide derivatives for use in treating a disorder in a patient expressing a high level of, and/or a constitutively active variant of USP8.

According to one of its objects, the present disclosure relates to a compound of general formula (I) : 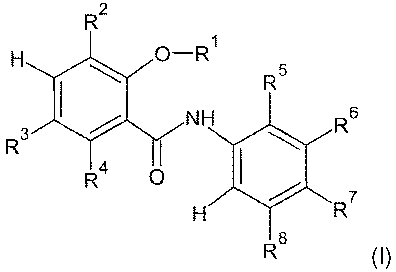 for use for treating an adrenocorticotropin hormone (ACTH)-dependent Cushing's syndrome,
wherein
- R¹ may be H or a -C(O)-R⁹ , wherein R⁹ may be a C₁-C₃ linear or branched, saturated or unsaturated, alkyl group, preferably -CH₃ or -CH₂-CH₂, and preferably -CH₃,
- R² may be H or a halogen, preferably I or Cl,
- R³ may be a halogen, preferably I or Cl,
- R⁴ and R⁸ are, independently, H or a halogen, preferably Cl,
- R⁵ may be H, a C₁-C₃, linear or branched, saturated or unsaturated, alkyl group, preferably CH₃, OH, or a halogen, preferably Ci,
- R⁶ may be H, a halogen, preferably Cl, or -C(O)-Ar, wherein Ar may be a C₆-C₁₀ aryl group, and preferably may be a phenyl group, Ar being optionally substituted with a C₁-C₃ linear or branched, saturated or unsaturated, alkyl group, preferably -CH₃ or - CH₂-CH₃, and preferably -CH₃,
- R⁷ may be H, a halogen, preferably Cl, a C₁-C₃, linear or branched, saturated or unsaturated, alkyl group, preferably -CH₃, -NO₂, or -B-Ar, wherein B may be O or -C(CN)-, Ar may be a C₆-C₁₀ aryl group, and preferably may be a phenyl group, Ar being optionally substituted with a halogen, preferably Cl,

wherein if R² is H, then at least one of R⁵, R⁶, R⁷ and R⁸ is not H,
or one of its pharmaceutically acceptable salts.

The compound may be a USP8 inhibitor. In some embodiments, the USP8 inhibitor may be a reversible inhibitor.

As shown in the Example Section, the inventors have surprisingly observed that the use of a cysteine-containing buffer as reaction buffer for selecting USP inhibitor, in particular USP8 inhibitor, allowed to identify a group of halogenated salicylanilide derivatives, in particular compounds of general formula (I), as selective and reversible inhibitor of the USP.

Advantageously, the compounds of formula (I) were able to selectively and reversibly inhibit the USP activity, such as USP8, with no or low toxicity.

Furthermore, the compounds of formula (I) were advantageously observed as inhibiting secretion of adrenocorticotropin hormone (ACTH) in a cultured pituitary cell line. Advantageously, the compounds of formula (I) were able to inhibit ACTH secretion by at least 30%, and more advantageously by at least 40% compared to a value of reference obtained in absence of inhibitor.

Furthermore, the compounds of formula (I) were advantageously observed as not affecting or as having reduced effect on cell viability of pituitary cells. Advantageously, the cell viability was maintained by at least 40%, and more advantageously by at least 60%, and more advantageously by about 90% or more when incubated in presence compounds of formula (I) compared to a value of reference obtained in absence of inhibitor.

In some embodiments, the ACTH-dependent Cushing's syndrome may be a Cushing's disease or a Cushing's syndrome associated with an ectopic ACTH secretion.

In some embodiments, R¹ may be H or -C(O)-CH₃.

In some embodiments, R² may be H, I or CI, and preferably may be I or Cl.

In some embodiments, R³ may be H, I or CI, and preferably may be I or Cl.

In some embodiments, R⁴ may be H or Cl.

In some embodiments, R⁵ may be H, OH, CH₃ or Cl

In some embodiments, R⁶ may be H, Cl, or C(O)-Phe-pCH₃.

In some embodiments, R⁷ may be H, Cl, CH₃, NO₂, O-Phe-*p*Cl or C(CN)-Phe-*p*Cl.

In some embodiments, R⁸ may be H or Cl.

In some embodiments, the compound may be such that:
- R¹ may be H or a -C(O)-CH₃,
- R² and R³ are, independently, I or CI,
- R⁴ and R⁸ are, independently, H or Cl,
- R⁵ may be H, -CH₃, OH, or Cl,
- R⁶ may be H, Cl, or C(O)-Phe-*p*Me,
- R⁷ may be H, Cl, CH₃, NO₂, O-Phe-*p*Cl or -C(CN)-Phe-*p*Cl.

In some embodiments, R² and R³ may both be the same halogen.

In some embodiments, the compound may be such that:
- R¹ may be H or a -C(O)-CH₃,
- R² and R³ are both I or are both CI,
- R⁴ and R⁸ are, independently, H or Cl,
- R⁵ may be H, -CH₃, OH, or CI,
- R⁶ may be H, Cl, or C(O)-Phe-*p*Me,
- R⁷ may be H, Cl, CH₃, or -C(CN)-Phe-*p*Cl.

In some embodiments, the compound may be one of: 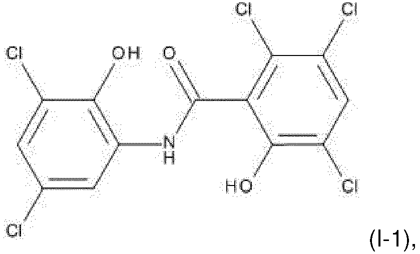 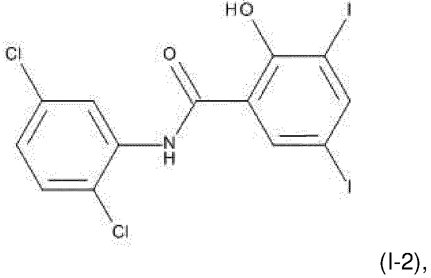 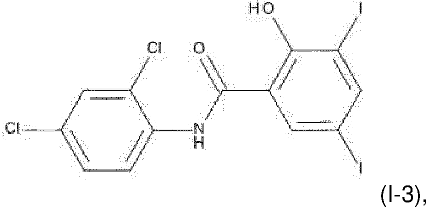 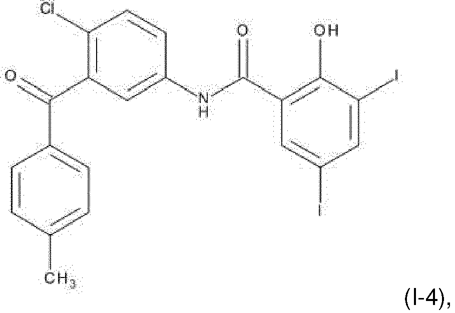 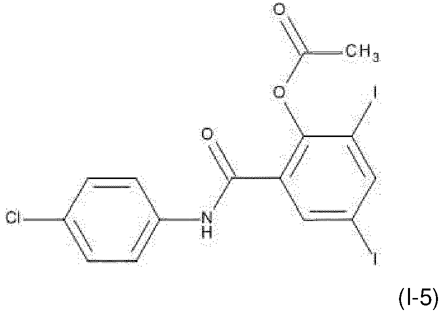 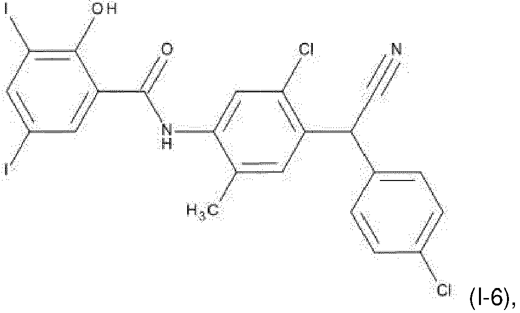 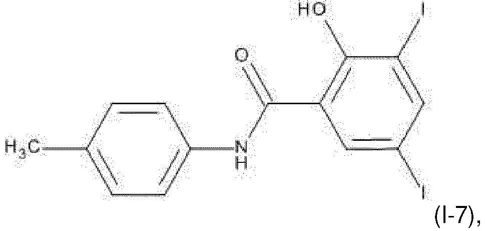 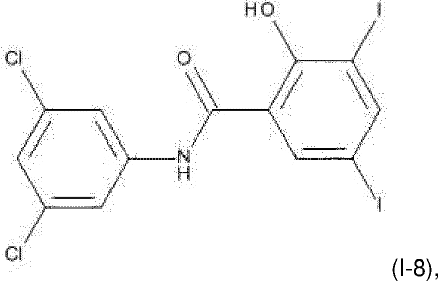 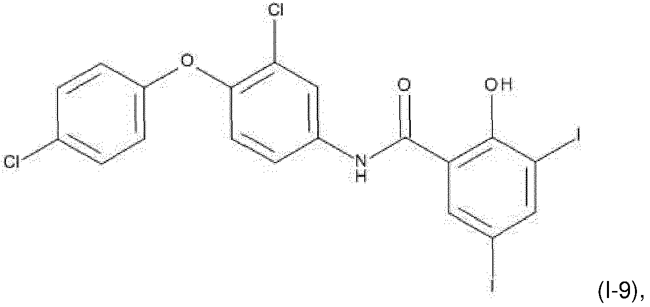 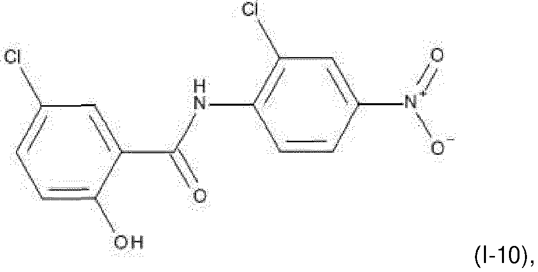 and

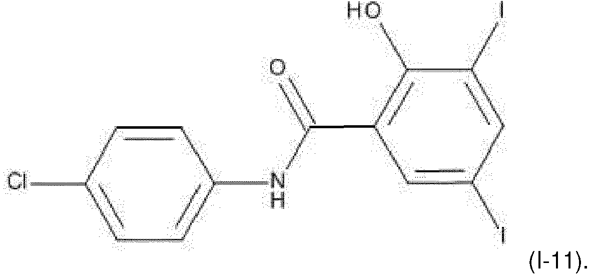

In some embodiments, the compound may be one of: (I-1), (I-2), (I-3), (I-4), I-5), (I-6), (I-7), and (I-8): 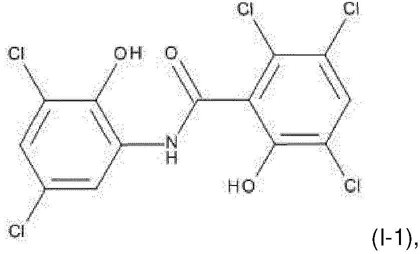 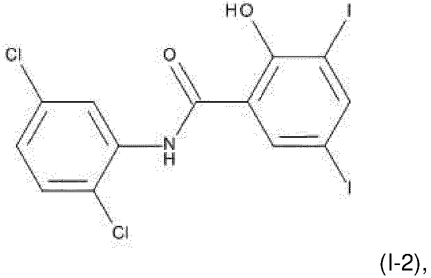 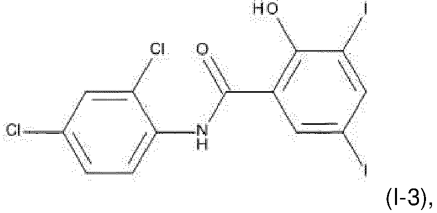 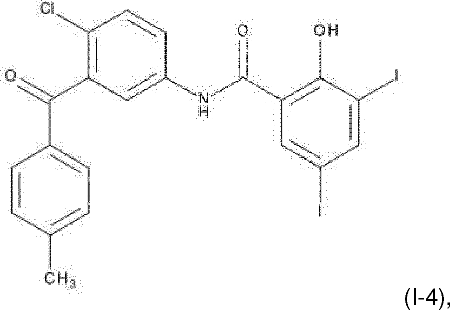 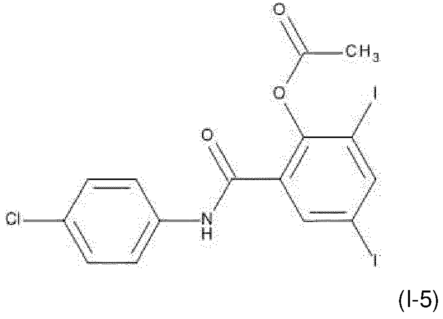 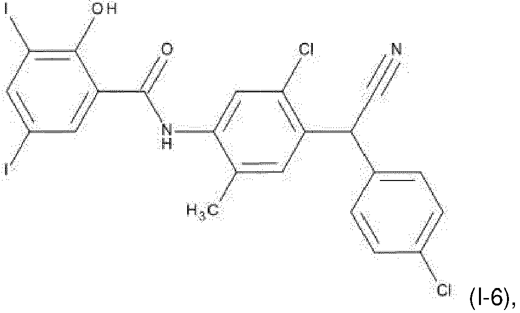 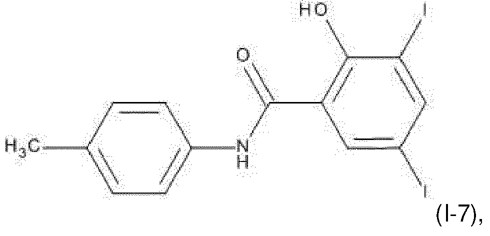 and

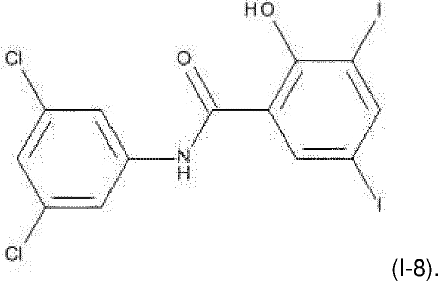

In some embodiments, the compound may be used in combination with an EGFR inhibitor. An EGFR inhibitor may be gefitinib, erlotinib, osimertinib, neratinib, cetuximab, panitumumab, dacomitinib, lapatinib, necitumumab, mobocertinib, or vandetanib, or combinations hereof. In some embodiments, an EGFR inhibitor may be gefitinib.

According to one of its objects, the present disclosure relates to a use of a cysteine-containing buffer for identifying a Ubiquitin Specific Protease (USP) inhibitor. The cysteine-containing buffer is used as a reaction buffer in which a catalytically active domain of USP, a substrate suitable for reporting the activity of the USP and a presumed USP inhibitor are contacted.

According to one of its objects, the present disclosure relates to a method for identifying a Ubiquitin Specific Protease (USP) inhibitor,
the method using at least:
- a catalytic domain of USP having a USP activity,
- a cysteine-containing buffer, and a
- substrate of said USP, said substrate being a reporter substrate or a substrate capable of being converted to a reporter molecule,
and
the method comprising at least the steps of:
a) contacting said catalytic domain of USP with a presumed USP inhibitor in said cysteine-containing buffer and said substrate in conditions suitable for the presumed USP inhibitor to bind to the catalytic domain of USP,
b) measuring an amount of said reporter substrate or of said reporter molecule obtained at step a), and
c) comparing the amount measured at step b) with a value of reference, the value of reference being obtained by contacting said catalytic domain with said substrate in absence of the presumed USP inhibitor,
wherein a deviation between the amount measured at step b) and the value of reference is indicative of an inhibition of the USP activity by said presumed USP inhibitor.

In some embodiments, the USP inhibitor may be a USP8 or a USP2 inhibitor. In some embodiments, the USP inhibitor may be a USP8 inhibitor.

In some embodiments, the cysteine containing buffer may comprise at least one of a phosphate buffer, a MES (morpholino-ethanesulfonic acid) buffer, a BIS-TRIS buffer, a citrate buffer, a TRIS-HCl buffer, a bicarbonate buffer, or a borate buffer.

In some embodiment, a suitable buffer may a Tris-HCl buffer.

In some embodiments, the cysteine containing buffer may comprise at least one of ovalbumin, bovine serum albumin, or human serum albumin.

In some embodiments, the cysteine may be present in the buffer in an amount ranging from about 3 mM to about 8 mM, from about 4 mM to about 7 mM, from about 5 mM to about 6 mM, and preferably is about 5.5 mM.

The cysteine may be a L- or D-cysteine.

In some embodiments, the catalytic domain of the USP may be provided as a full-length USP or as a fragment of USP containing said catalytic domain. A suitable fragment of USP may be any truncated form of the full-length USP provided it contains an active catalytic domain.

In some embodiments, a substrate may be ubiquitin-Rhodamine 110 or ubiquitin-7-amido-4-methylcoumarin. It may also be any kind of di-, tri, tetra or multi-ubiquitin chains associated or not with a fluorescent or a chemi-luminescent molecule, or associated with an enzymatic activity (such as ubiquitin-based proluciferin, Ub-Lys-TAMRA-Gly, etc.).

According to another object, the present disclosure relates to a method for treating an ACTH-dependent Cushing's syndrome, the method comprising the administration of a therapeutically effective amount of a compound of formula (I) to a patient in need thereof.

According to another object, the present disclosure relates to a compound of formula (I) for treating an ACTH-dependent Cushing's syndrome in a patient in need thereof.

According to another object, the present disclosure relates to a compound of formula (I) for use in the manufacture of a medicament for treating an ACTH-dependent Cushing's syndrome in a patient in need thereof.

### [DESCRIPTION OF THE FIGURES]

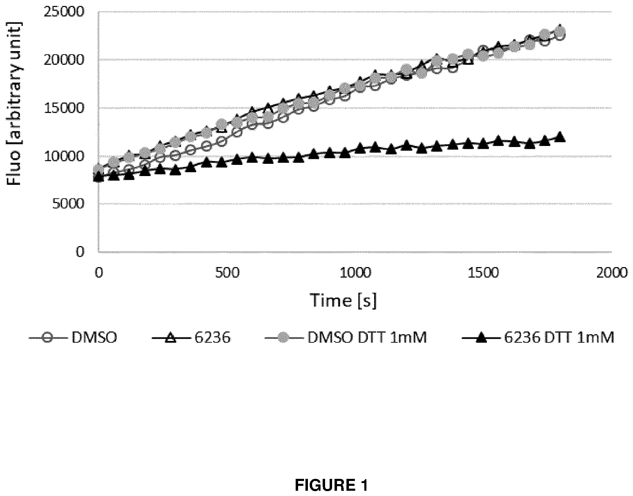

**Figure 1****:** illustrates the USP8 catalytic activity monitored in the presence (filled circles) or absence (empty circles) of dithiothreitol (DTT). PCR6236 (indicated 6236 - triangles) is a strong inhibitor of USP8 in the presence of DTT (filled triangles) while not in the absence of DTT (here replaced by L-Cysteine - empty triangles). DMSO (0.5%) is used as the bio-inactive control resulting in maximal catalytic activity of USP8 CD (USP8 catalytic domain).

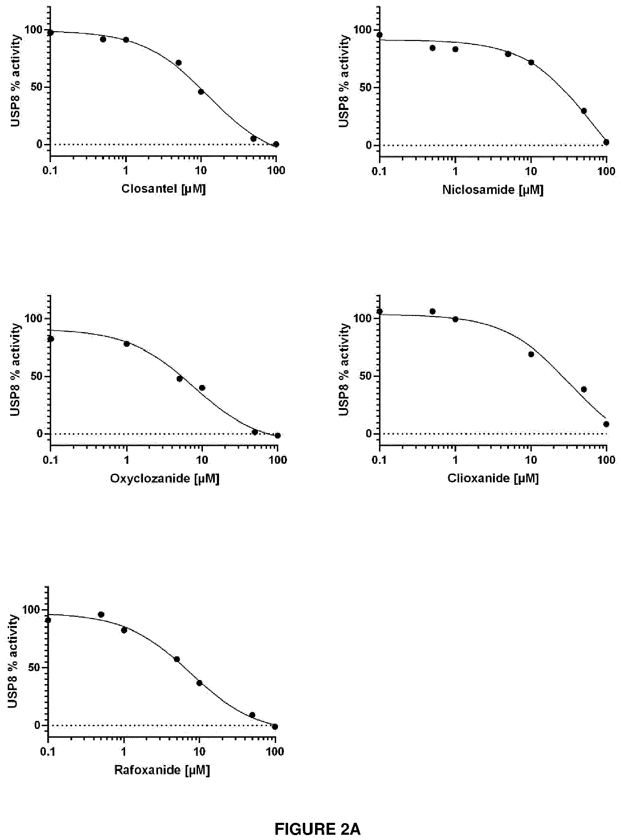

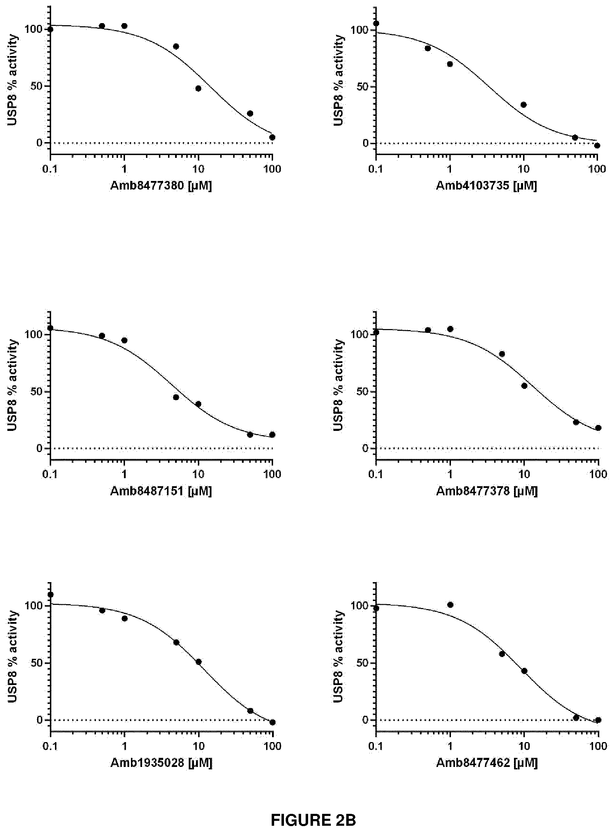

**Figure 2** **(A & B):** illustrates the inhibition of USP8 Catalytic activity by closantel and active analogs (IC₅₀ determination). USP8 FL (USP8 Full Length) was incubated at a concentration of 75 nM in the presence of Ub-Rho (UBIQUIGENT ref. 60-0117-050) at a final concentration of 0.1 µM, in conditions ensuring linearity of enzyme activity throughout the assay in a specific buffer. Compounds were added to the reaction mix at different doses just before the substrate. The enzymatic reaction kinetics were monitored by measuring fluorescence elicited by the hydrolysis of Ub-Rho over 30 min.

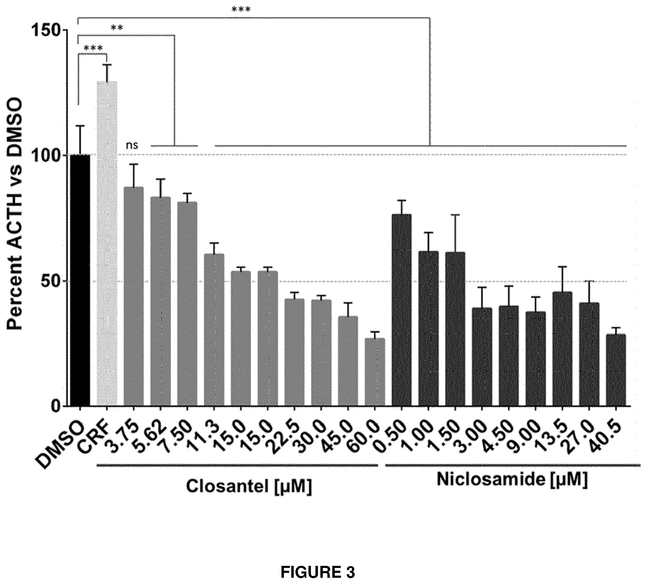

**Figure 3****:** illustrates a decrease of the ACTH concentration in the supernatant from AtT-20 pituitary cells treated with closantel or niclosamide. ACTH release (percent comparison to DMSO control). Briefly, 12,500 AtT-20 cells per well were seeded in a 96-well plate. After 24 hours exposure, cells were treated with each compound at the indicated final concentration, or with DMSO alone (control condition compound solvent) or with corticotropin releasing factor (indicated CRF) at 100 nM. After a 24-hour treatment, cell supernatants were collected and stored at -80°C, prior to quantification of ACTH concentration using ACTH chemiluminescent E!A kit (Phoenix Pharmaceuticals, Inc.). Determination of ACTH concentrations was based on the best-fitting ACTH standard curve and P values were calculated using one-way ANOVA, using Prism 9 software (GraphPad Software, Inc.) ns (P=0.12). **P<0.002. ***P< 0.001. All compounds were tested in technical triplicates. One representative experiment out of three is presented.

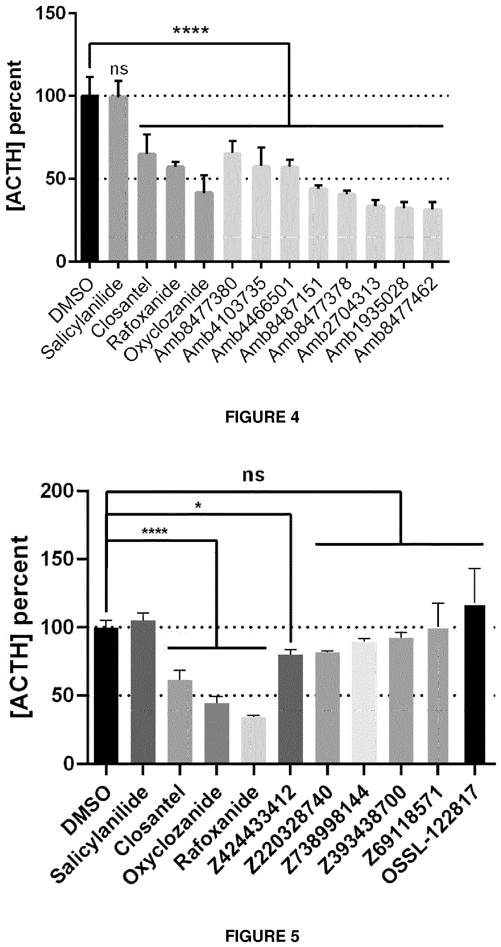

**Figure 4****:** illustrates a decrease of ACTH secretion by AtT-20 cells after 24-hour treatment of halogenated salicylanilides. 12,500 AtT-20 cells per well were seeded in a 96-well plate. After 24 hours, cells were treated with salicylanilide backbone or halogenated salicylanilide derivatives (corresponding chemical formula are indicated in Table 1) at 25 µM final concentration, or with DMSO 0.05% (compound solvent). After a 24-hour treatment, cell supernatants were collected and stored at -80°C, prior to quantification of ACTH concentration using ACTH chemiluminescent EIA kit (Phoenix Pharmaceuticals, Inc.). Determination of ACTH concentrations was based on the best-fitting ACTH standard curve and P values were calculated by ordinary one-way ANOVA using Prism 6 software (GraphPad Software, Inc), ns (not significant), **** P < 0.0001. Compounds were tested in technical triplicates.
**Figure 5****:** illustrates the ACTH secretion by AtT-20 cells after 24-hour treatment with closantel or analogs. 12,500 AtT-20 cells per well were seeded in a 96-well plate. After 24 hours, cells were treated with compounds (corresponding chemical formula are indicated in Table 1) at 25 µM final concentration, or with DMSO alone (compound solvent). After a 24-hour treatment, cell supernatants were collected and stored at -80°C, prior to quantification of ACTH concentration using ACTH chemiluminescent EIA kit (Phoenix Pharmaceuticals, Inc.). Determination of ACTH concentrations was based on the best-fitting ACTH standard curve and P values were calculated by ordinary one-way ANOVA using Prism 6 software (GraphPad Software, Inc), ns (not significant), *P<0.O5, ***P<0,0001. Compounds were tested in technical triplicates.

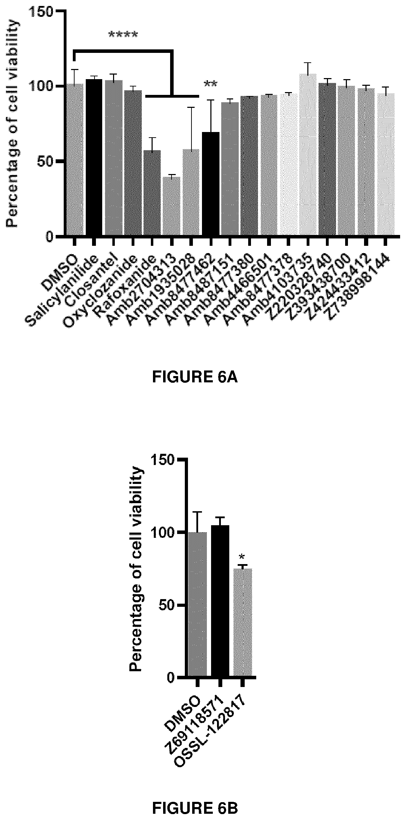

**Figure 6** **(A & B):** illustrates the viability assay of the AtT-20 murine cells after 24-hour compound exposure, reported to DMSO control levels. The PrestoBlue^{™} Cell Viability Reagent, quickly reduced to a fluorescent product by metabolically active cells, provides a quantitative measure of viability. Mean ± SD. **P<0.01; ***P<0.001; **** P<0.0001. Otherwise, P>0.5 (non-significant). Ordinary one-way ANOVA.

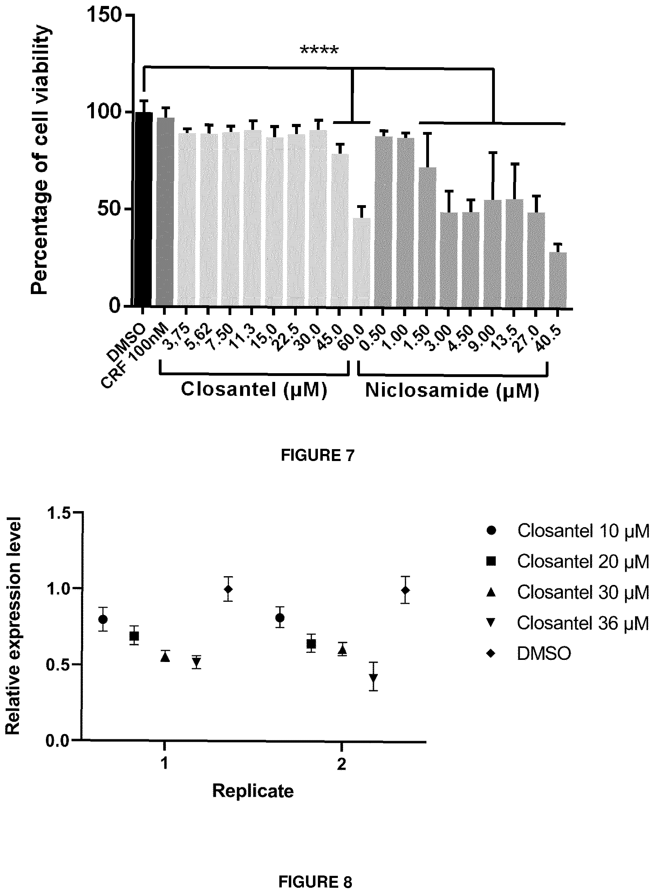

**Figure 7****:** illustrates a viability assay of the AtT-20 murine cells after 24-hour compound exposure as compared to 0.5 % DMSO control (100%). The PrestoBlue^{™} Cell Viability Reagent, quickly reduced to a fluorescent product by metabolically active cells, provides a quantitative measure of viability and cytotoxicity. Mean ± SD. CRF = corticotropin releasing factor. Ns (non-significant). * P<0.033. ** P<0.002. *** P<0.001.
**Figure 8****:** illustrates a POMC encoding gene expression assay in presence or absence of different concentrations (10, 20, 30 and 36 µM) of closantel in two independent experiments (biological replicates). Each dot represents the amount of POMC encoding transcripts normalized to the amount of transcripts encoding the housekeeping gene HPRT and then compared to the DMSO control condition. Mean of technical triplicates with errors bars.

### [DETAILED DESCRIPTION]

### Definitions

It must be noted that as used herein and in the appended claims, the singular forms "**a**", "**an**", and "**the**" include plural referents unless the context clearly dictates otherwise. Thus, for example, reference to "an antibody" includes a plurality of such antibodies, and so forth.

The terms **"about"** or **"approximately"** as used herein refer to the usual error range for the respective value readily known to the skilled person in this technical field. Reference to **"about"** a value or parameter herein includes (and describes) embodiments that are directed to that value or parameter per se. In some embodiments, the term "about" refers to ±10% of a given value. However, whenever the value in question refers to an indivisible object, such as a molecule or other object that would lose its identity once subdivided, then "about" refers to ±1 of the indivisible object.

It is understood that aspects and embodiments of the present disclosure described herein include **"having," "comprising," "consisting of,"** and **"consisting essentially of"** aspects and embodiments. The words **"have"** and **"comprise,"** or variations such as **"has," "having," "comprises,"** or **"comprising,"** will be understood to imply the inclusion of the stated element(s) (such as a composition of matter or a method step) but not the exclusion of any other elements. The term **"consisting of"** implies the inclusion of the stated element(s), to the exclusion of any additional elements. The term **"consisting essentially of"** implies the inclusion of the stated elements, and possibly other element(s) where the other element(s) do not materially affect the basic and novel characteristic(s) of the disclosure. It is understood that the different embodiments of the disclosure using the term **"comprising"** or equivalent cover the embodiments where this term is replaced with **"consisting of"** or **"consisting essentially of".**

The expression **"USP inhibitor"** intends to refer to a compound able to bind to a ubiquitin specific protease and to prevent the enzyme-catalyzed reaction. Inhibition may be reversible or irreversible. The USP inhibitor Closantel is shown to be a reversible inhibitor. Further, the compounds of general formula (I) are selective to USP, such as USP8 and the very closely related enzyme USP2. A selective enzyme inhibitor blocks the action of the enzyme by binding to the free enzyme, preventing the substrate from binding and thus preventing the enzyme from acting on that substrate. A selective enzyme inhibitor for a given enzyme has a higher potency for inhibiting this enzyme than for other enzymes.

The expression **"adrenocorticotropin hormone (ACTH)-dependent Cushing's syndrome"** intends to refer to the morbidities resulting from excessive secretion of cortisol which can be observed In Cushing's disease patients or induced by other ectopic cancerous cells secreting ACTH.

The expression **"Cushing's disease"** intends to refer to a disease in which the presence of a microadenoma (or macroadenoma in very rare cases) in the pituitary gland causes a deregulated secretion of adrenocorticotropic hormone (ACTH), resulting in a deregulated release of cortisol and adrenal androgens by the adrenal gland cortex.

Patients suffering from Cushing's syndrome or from Cushing's disease may suffer from central obesity, skin atrophy, muscle wasting, cardiovascular disease, diabetes, metabolic syndrome, osteoporosis, disturbed mood and impaired reproductive function. These morbidities are all related to an excess of corticoid hormones and define the Cushing's syndrome.

As used herein, the term "**alkyl**" refers to a linear or branched, saturated or unsaturated alkyl group having 1 to 3 carbon atoms, such as methyl, ethyl, n-propyl, or isopropyl. A designation such as "C₁-C₃ alkyl" refers to an alkyl radical containing from 1 to 3 carbon atoms.

The term "**halogen**" intends to refer to a halogen atom such as a fluorine, a chlorine, a bromine or an iodine atom, and in particular a iodine and a chlorine atom.

An "**aryl group (Ar)**" intends to refer to an aromatic ring comprising from 1 to 3 aromatic rings, optionally fused, and comprising from 6 to 20 carbon atoms, or from 6 to 10 carbon atoms. As examples of aryl group, one may mention a group from phenyl (Phe), naphthyl, or anthryl radical, and all the aromatic rings comprising one or more heteroatoms chosen from O, N and S, such as, for example, pyridine, thiophene, pyrrole, furan and quinoline. An aryl group may be substituted with halogen(s) and/or with C₁-C₃ alkyl group(s). "**Arylene group**" is understood to mean an aryl radical, as defined above, which is bivalent.

When an aryl group is a phenyl group bearing one or more substituent(s), the letter "*o*", "*m*" and "*p*" associated with the substituent(s), such as *p*Cl refers the position of the substituent in ortho, meta or para.

"**Pharmaceutically acceptable salt**" as used herein refers to acid addition salts or base addition salts of the compounds in the present disclosure. A pharmaceutically acceptable salt is any salt which retains the activity of the parent compound, and which may be administered to a patient in a context of a pharmaceutical use. Pharmaceutically acceptable salts include, but are not limited to, metal complexes and salts of both inorganic and carboxylic acids. Pharmaceutically acceptable salts may include metal salts such as sodium, potassium, aluminum, calcium, iron, magnesium, manganese and complex salts. In addition, pharmaceutically acceptable salts may include, but are not limited to, acid salts such as acetic, aspartic, benzoic, bicarbonic, bisulfuric, bitartaric, butyric, calcium edetate, citric, edetic, formic, fumaric, gluconic, glutamic, glycolic, hydrobromic, hydrochloric, hydrochloride, maleic, malic, malonic, mandelic, nitric, oxalic, pantothenic, phosphoric, monohydrogen phosphoric, dihydrogen phosphoric, phthalic, propionic, salicylic, stearic, succinic, sulfonic, tannic, tartaric, and the like. Base addition salts include those derived from inorganic bases such as ammonium and alkali and alkaline earth metal hydroxides, carbonates, bicarbonates, and the like, as well as salts derived from basic organic amines such as aliphatic and aromatic amines, aliphatic diamines, hydroxy alkamines, and the like. Such bases useful in preparing the salts of this invention thus include ammonium hydroxide, potassium carbonate, sodium bicarbonate, calcium hydroxide, methylamine, diethylamine, ethylenediamine, cyclohexylamine, ethanolamine and the like.

As used herein, terms "patient", **"subject" "recipient"** or **"individual"** are used interchangeably and intends to refer to a mammal, preferably a human, or a human child, which is afflicted with, or has the potential to be afflicted with one or more diseases and conditions described herein.

As used herein, a **"therapeutically effective amount"** refers to an amount of a compound of the present disclosure effective to prevent or treat the symptoms of particular disorder.

As used herein, the terms **"treat", "treating", "treatment", "therapy"** and the like refer to the administration or consumption of a compound as disclosed herein with the purpose to cure, heal, alleviate, relieve, alter, remedy, ameliorate, improve, or affect a disease or a disorder, the symptoms of the condition, or to prevent or delay the onset of the symptoms, complications, or otherwise arrest or inhibit further development of the disorder in a statistically significant manner. Also, as used herein, in the context of the present disclosure, the terms **"treat", "treatment"** and the like refer to relief from or alleviation of pathological processes mediated by USP8. In the context of the present disclosure, insofar as it relates to any of the other conditions recited herein, the terms **"treat", "treatment",** and the like refer to relieving or alleviating one or more symptoms associated with such condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention. All publications mentioned herein are incorporated herein by reference to disclose and describe the methods and/or materials in connection with which the publications are cited.

The list of sources, ingredients, and components as described hereinafter are listed such that combinations and mixtures thereof are also contemplated and within the scope herein.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

All lists of items, such as, for example, lists of ingredients, are intended to and should be interpreted as Markush groups. Thus, all lists can be read and interpreted as items "selected from the group consisting of' the list of items "and combinations and mixtures thereof."

Referenced herein may be trade names for components including various ingredients utilized in the present disclosure. The inventors herein do not intend to be limited by materials under any particular trade name. Equivalent materials (e.g., those obtained from a different source under a different name or reference number) to those referenced by trade name may be substituted and utilized in the descriptions herein.

### Compounds

The compounds of the disclosure are halogenated salicylanilide derivatives.

A compound of the disclosure may be of general formula (I): 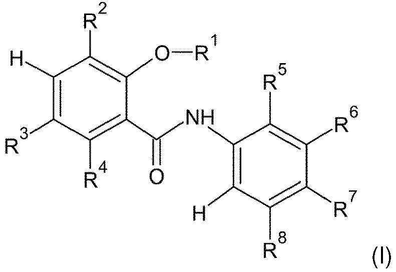 wherein
- R¹ may be H or a -C(O)-R⁹ , wherein R⁹ may be a C₁-C₃ linear or branched, saturated or unsaturated, alkyl group,
- R² may be H or a halogen,
- R³ may be a halogen,
- R⁴ and R⁸ are, independently, H or a halogen,
- R⁵ may be H, a C₁-C₃, linear or branched, saturated or unsaturated, alkyl group, OH, or a halogen,
- R⁶ may be H, a halogen, or -C(O)-Ar, wherein Ar may be a C₆-C₁₀ aryl group, Ar being optionally substituted with a C₁-C₃ linear or branched, saturated or unsaturated, alkyl group,
- R⁷ may be H, a halogen, a C₁-C₃, linear or branched, saturated or unsaturated, alkyl group, -NO₂, or -B-Ar , wherein B may be O or -C(CN)-, Ar may be a C₆-C₁₀ aryl group, Ar being optionally substituted with a halogen,

wherein if R² is H, then at least one of R⁵, R⁶, R⁷ and R⁸ is not H,
or one of its pharmaceutically acceptable salts .

In some embodiments, a suitable pharmaceutical salt may be a sodium, an aluminium, a potassium, a calcium, a magnesium, or a manganese salt. In some embodiments, a suitable pharmaceutical salt may be a sodium salt.

In some embodiments, R¹ may be H.

In some embodiments, R¹ may be -C(O)-R⁹. R⁹ may be a C₁-C₃ linear or branched, saturated or unsaturated, alkyl group. R⁹ may be -CH₃ or -CH₂-CH₃. R⁹ may be - CH₃.

In some embodiments, R¹ may be H or -C(O)-CH₃.

In some embodiments, R² may be H.

In some embodiments, R² may be a halogen. A halogen may be I or Cl.

In some embodiments, R² may be H, I or Cl. In some embodiments, R² may be I or Cl.

In some embodiments, R³ may be a halogen. In some embodiments, R³ may be I or Cl,

In some embodiments, R⁴ may be H.

In some embodiments, R⁴ may be a halogen. A halogen may be Cl or I. A halogen may be Cl.

In some embodiments, R⁴ may be H or Cl. In some embodiments, R⁴ may be Cl.

In some embodiments, R⁵ may be H.

In some embodiments, R⁵ may be a C₁-C₃, linear or branched, saturated or unsaturated, alkyl group. An alkyl group may be -CH₂-CH₃ or -CH₃. An alkyl group may be -CH₃.

In some embodiments, R⁵ may be OH.

In some embodiments, R⁵ may be a halogen. A halogen may be I or CI. A halogen may be Cl.

In some embodiments, R⁵ may be CH₃, OH, or Cl.

In some embodiments, R⁶ may be H.

In some embodiments, R⁶ may be a halogen. A halogen may be Cl or I. In some embodiments, a halogen may be Cl.

In some embodiments, R⁶ may be -C(O)-Ar, wherein Ar may be a C₆-C₁₀ aryl group, optionally substituted with a C₁-C₃ linear or branched, saturated or unsaturated, alkyl group. In some embodiments, Ar may be substituted with -CH₂ or -CH₂-CH₃. In some embodiments, Ar may be substituted with -CH₃. Ar may be a phenyl group. Ar may be a phenyl group, optionally substituted with an C₁-C₃ alkyl group. The phenyl group may be substituted by a C₁-C₃ alkyl group in *ortho* (*o*)*, meta* (*m*) or *para* (*p*) position. In some embodiments, Ar may be a phenyl group (Phe) substituted with -CH₃. In some embodiments, Ar may be a phenyl group (Phe) substituted with -CH₃ in *para* position (Phe-*p*CH₃).

In some embodiments, R⁶ may be H, Ci, or C(O)-Phe-pCH₃.

In some embodiments, R⁷ may be H.

In some embodiments, R⁷ may be a halogen. A halogen may be Cl or I. A halogen may be Cl.

In some embodiments, R⁷ may be a C₁-C₃, linear or branched, saturated or unsaturated, alkyl group. R⁷ may be -CH₂ or -CH₂-CH₃. R⁷ may be -CH₃.

In some embodiments, R⁷ may be -NO₂.

In some embodiments, R⁷ may be -B-Ar. B may be O or -C(CN)-. Ar may be a C₆-C₁₀ aryl group, optionally substituted with a halogen. In some embodiments, Ar may be substituted with Cl or I. In some embodiments, Ar may be substituted with Cl. Ar may be a phenyl group. Ar may be a phenyl group, optionally substituted with halogen. The phenyl group may be substituted by a halogen in *ortho* (*o*)*, meta* (*m*) or *para* (*p*) position. In some embodiments, Ar may be a phenyl group (Phe) substituted with I or Cl. Ar may be a phenyl group (Phe) substituted with Cl. In some embodiments, Ar may be a phenyl group (Phe) substituted with Cl in *para* position (Phe-*p*Cl).

In some embodiments, R⁷ may be O-Phe-*p*Cl or C(CN)-Phe-*p*Cl.

In some embodiments, R⁷ may be H, Cl, CH₃, NO₂, O-Phe-*p*Cl or C(CN)-Phe-pCl.

In some embodiments, R⁸ may be H.

In some embodiments, R⁸ may be a halogen. A halogen may be Cl or I.

In some embodiments, R⁸ may be H or Cl.

In the compound of general formula (I), when R² is H, then at least one of R⁵, R⁶, R⁷ and R⁸ is not H.

In some embodiments, a compound of general formula (I) may be such that:
- R¹ may be H or a -C(O)-CH₃,
- R² and R³ are, independently, I or Cl,
- R⁴ and R⁸ are, independently, H or Cl,
- R⁵ may be H, -CH₃, OH, or CI,
- R⁶ may be H, CI, or C(O)-Phe-*p*Me,
- R⁷ may be H, Cl, CH₃, O-Phe-pCI or -C(CN)-Phe-pCl.

In some embodiments, R² and R³ may both be the same halogen.

In some embodiments, a compound of general formula (I) may be such that:
- R¹ may be H or a -C(O)-CH₃,
- R² and R³ may be both I or may be both Cl,
- R⁴ and R⁸ are, independently, H or Cl,
- R⁵ may be H, -CH₃, OH, or Cl,
- R⁶ may be H, Cl, or C(O)-Phe-*p*Me,
- R⁷ may be H, Cl, CH₃, -O-Phe-*p*Cl or -C(CN)-Phe-*p*Cl.

In some embodiments, a compound of general formula (I) may be such that:
- R¹ may be H or a -C(O)-CH₃,
- R² and R³ may be both I or may be both Cl,
- R4 and R8 are, independently, H or Cl,
- R5 may be H, -CH3, OH, or Cl,
- R6 may be H, Cl, or C(O)-Phe-*p*Me,
- R7 may be H, Cl, CH3, or -C(CN)-Phe-*p*Cl.

In some embodiments a compound of general formula (I) may be such that:
- R¹ may be H,
- R² and R³ may be both I,
- R⁴ may be H,
- R⁵ may be H, -CH₃, or CI,
- R⁶ may be H, CI, or C(O)-Phe-*p*Me,
- R⁷ may be H, Cl, CH₃, -O-Phe-*p*Cl or -C(CN)-Phe-*p*Cl, and
- R⁸ may be H or CI.

In some embodiments a compound of general formula (I) may be such that:
- R¹ may be H,
- R² and R³ may be both I,
- R⁴ may be H,
- R⁵ may be H, -CH₃, or Cl,
- R⁶ may be H, Cl, or C(O)-Phe-*p*Me,
- R⁷ may be H, Cl, CH₃, or -C(CN)-Phe-*p*Cl, and
- R⁸ may be H or Cl.

In some embodiments, a compound of general formula (I) may be such that:
- R¹ may be H,
- R², R³, R⁴, R⁶ and R⁸ may be Cl,
- R⁷ may be H,
- R⁵ may be OH,

In some embodiments, a compound of general formula (I) may be such that:
- R¹, R², R⁴, R⁶ and R⁸ may be H,
- R³ and R⁵ may be Cl, and
- R⁷ may be NO₂.

In some embodiments, a compound of general formula (I) may be such that:
- R¹ may be -C(O)-CH₃,
- R² and R³ may be both I,
- R⁴, R⁵, R⁶ and R⁸ may be H,
- R⁷ may be Cl.

In some embodiments, a compound of general formula (I) may be any one of: 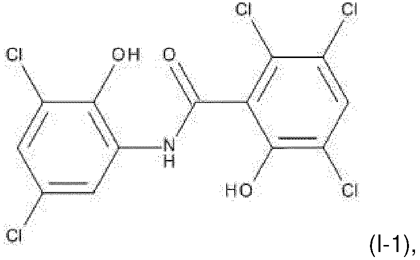 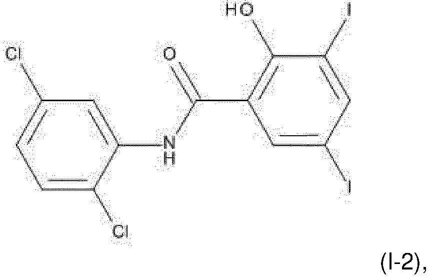 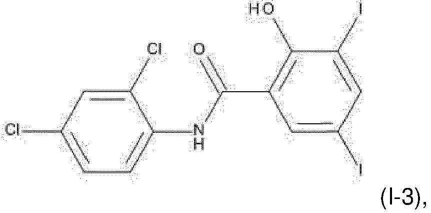 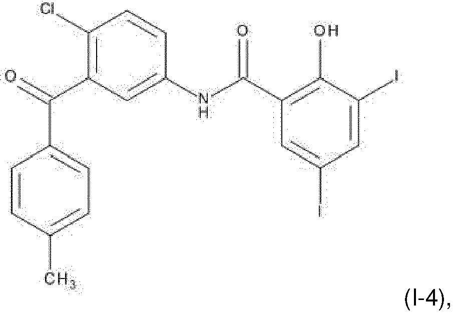 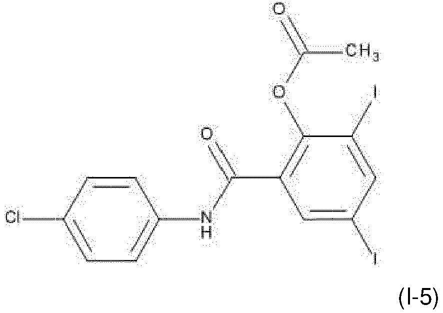 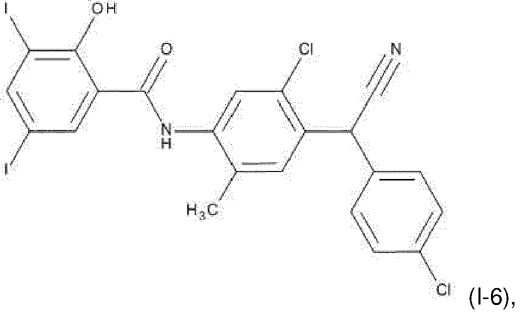 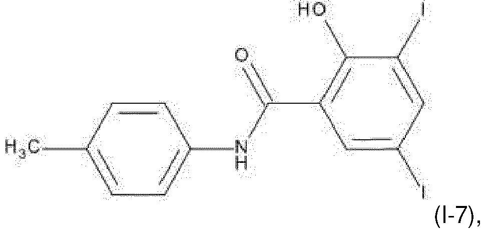 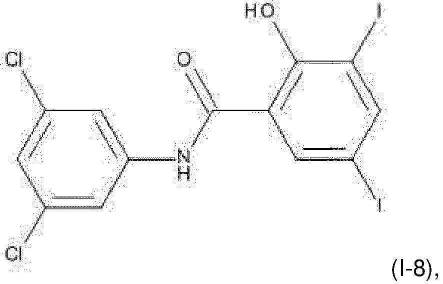 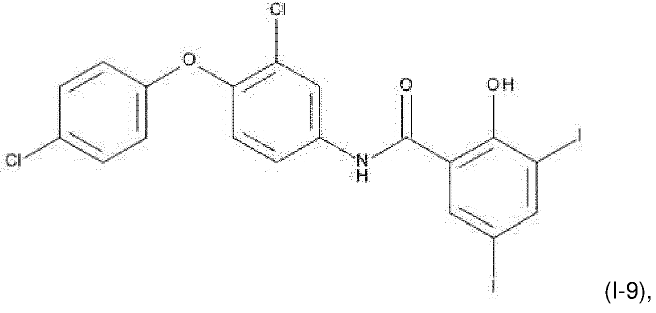 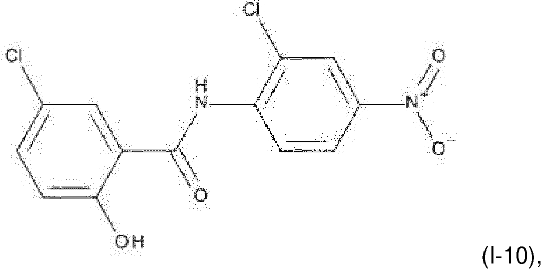 and

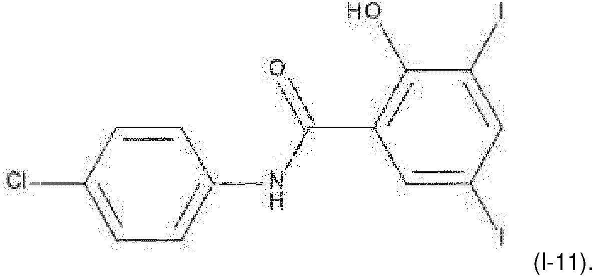

In some embodiments, a compound of general formula (I) may be any one of: (I-1), (I-2), (I-3), (I-4), I-5), (I-6), (I-7), and (I-8): 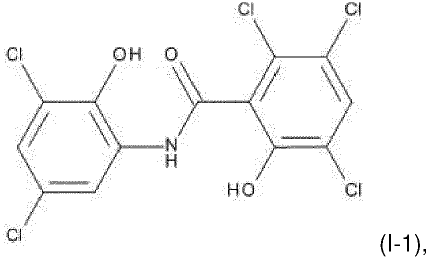 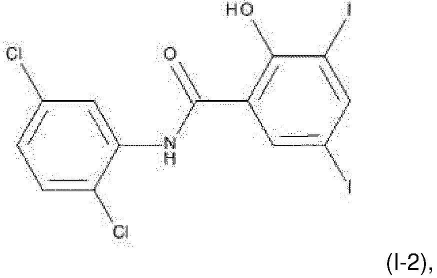 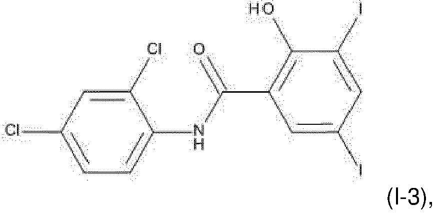 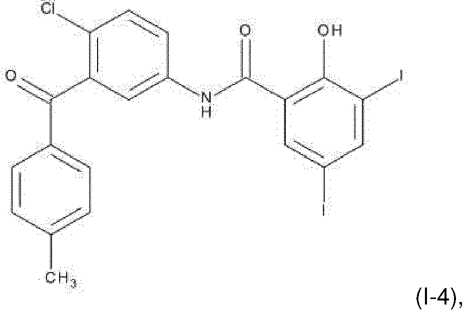 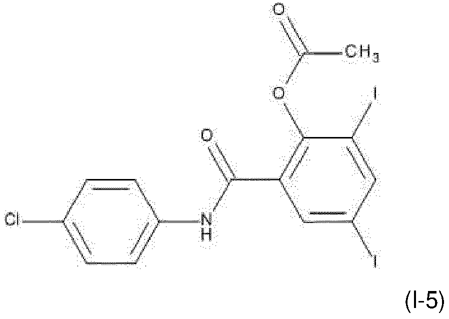 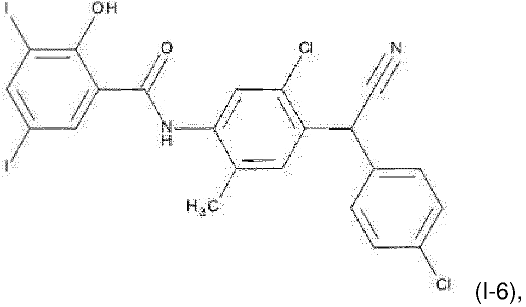 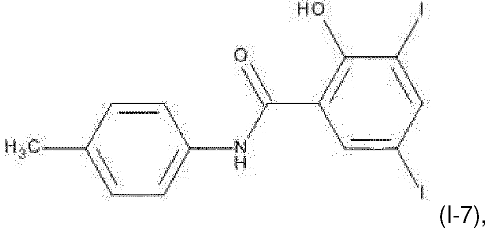 and

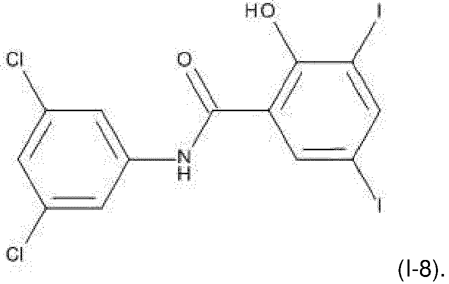

The compounds of general formula (I) are halogenated salicylanilide derivatives which can be prepare according to any chemical synthesis protocol known in the field.

As example of synthesis schemes which can be used for preparing the compounds disclosed herein, one may mention the methods disclosed in Vinsova et al., Molecules. 2007;12(1):1-12, Sharma & Anand, Chap. 9 - Salicylanilides, Pharmacochemistry Library, Elsevier, Vol. 25, 1997, 239-257, Baranyai et al., Eur J Med Chem. 2017;133:152-173, Chen et al., Eur J Med Chem. 2016;117:70-84, Kadam, et al., J Chem, Biol Phys Sci, 2014, 44. 918-926, or in CN109851526A. Those methods can be easily by one skilled in the art to prepare the compounds disclosed herein.

Alternatively, the compounds of general formula (I) may be commercially available.

For example, closantel (MCE HY-17596, compound (I-6)), niclosamide (MCE HY-B0497, compound (I-9), oxyclozanide (MCE HY-17594, compound (I-1), rafoxanide (MCE HY-17598, compound (I-8), can be obtained from MedChemExpress.

Also, Amb8477380 (compound (I-7)), Amb4103735 (compound (I-4)), clioxanide (Amb4466501, compound (I-5)), Amb8487151 (compound (I-3)), Amb8477378 (compound (I-2)), Amb1935028 (compound (I-10)), and Amb8477462 (compound (I-11)) can be obtained from Ambinter.

### Uses

Compounds of general formula (I) are inhibitor of USP8.

Compounds of general formula (I) are reversible inhibitor of USP8.

Compounds of general formula (I) are selective, reversible, inhibitor of USP8.

The USP8 activity is a deubiquitinase activity resulting in the hydrolysis of isopeptide bonds and the removal of ubiquitin from ubiquitinated proteins. The measure of the USP8 activity may be carried out according to methods known in the field, or as detailed thereafter or in the Example section.

The USP8 activity may be measured either directly by a measure of the hydrolysis activity represented by the remaining amount of a reporter substrate hydrolyzed by the enzyme or by the amount of a reporter molecule resulting from the hydrolysis of a substrate, at a given time after contacting the substrate and the enzyme. The measured amount is indicative of the enzyme activity.

The USP8 activity may be measured indirectly, in a cell system, by the measure of an amount of a biomarker, for example ACTH, the accumulation of which or the disappearance of which, is depending on the USP activity. For example, the biomarker may be the secretion of ACTH.

A measure may be carried out at a given time, or at various points of time.

A measure may be carried out in presence or in absence of an inhibitor.

The measured amount is compared to a value of reference.

A value of reference may be the amount of the reporter substrate or of the reporter molecule in absence of enzyme.

Alternatively, a value of reference may be the amount of the reporter substrate or of the reporter molecule in presence of an enzyme and in absence of a compound of the disclosure measured at a given time, or at various points of time.

The value of reference to be considered is selected according to the information sought, i.e., the enzyme activity of USP8 per se or the level of enzyme inhibition achieved by an inhibitor.

In some embodiments, the USP8 activity is measured according to the method disclosed in the Example section.

In case of a measure of a biomarker in a cell system, a value of reference may the amount of biomarker in absence of inhibitors.

In some embodiments, the potency of an inhibitor is measured according to the method disclosed in the Example section.

The inhibition of an enzyme activity in presence of a compound of general formula (I) may be partial or total.

A total inhibition is obtained when about 100% of the enzyme activity is inhibited.

A partial inhibition is obtained when less than 100% of the enzyme activity is inhibited.

A suitable inhibition of USP8 for the invention may be achieved when at least about 30% of the enzyme activity is inhibited.

In some embodiments, a compound of general formula (I) may inhibit the hydrolysis activity of the USP8 by at least about 30%, 40, 50, 55, 60, 65, 70, 75, 80, 85, 90, 95, about 99%, or 100% relative the USP8 activity in absence of said inhibitor.

In some embodiments, a compound of general formula (I) may inhibit an ACTH secretion by cells, for example by At-20 cells, by at least about 30%, 40, 50, 55, 60, 65, 70, 75, 80, 85, 90, 95, about 99%, or 100% relative the ACTH secretion in absence of said inhibitor.

Compounds of general formula (I) may be for use for treating a disorder resulting from a high level of USP8, and/or a constitutively active variant of USP8.

The uses according to the disclosure are in a patient in need thereof.

The term *"variant"* used with regard to USP8 intends to refer to a USP8 protein highly similar a USP8 originating from the gene coding for USP8 (including isoforms resulting from alternate splicing) but displaying one or several mutations (nucleotide(s) deletion, substitution, or addition) in the gene coding sequence. "Constitutive active variants" intends to refer to variants of USP8 for which the enzyme activity is not regulated, in particular inhibited, by naturally occurring cell process, such as interaction with 14-3-3.

Compounds of general formula (I) may be for use in treating disorders for which a reduction of the activity of USP8 is beneficial. A reduction of the enzyme activity is appreciated with respect to the activity of the USP8 before administration of a compound of general of formula (I) to a patient in need thereof.

Compounds of general formula (I) may be for use in treating disorders induced by gain-of-function variants of USP8.

The expression "gain-of-function variant" intends to refer to a USP8 variant which has an enhanced enzyme activity, or which is a constitutively active enzyme.

A gain-of-function variant of USP8 may be a constitutively active form of USP8.

Compounds of general formula (I) may be for use for reducing ACTH secretion.

Compounds of general formula (I) may be for use for treating a disorder associated with an excess of ACTH secretion.

Compounds of general formula (I) may be for use for treating an adrenocorticotropin hormone (ACTH)-dependent Cushing's syndrome.

Compounds of general formula (I) may be for use in treating a Cushing's syndrome.

Compounds of general formula (I) may be for use in treating a Cushing's disease.

Compounds of general formula (I) may be for use in treating a Cushing's syndrome associated with an ectopic ACTH secretion.

Compounds of general formula (I) may be for use in treating a Cushing's disease resulting from a pituitary tumor, extra pituitary tumor cells secreting ACTH, or adrenocortical tumor secreting corticoid hormones.

Compounds of general formula (I) may be for use in preventing and/or treating a Cushing's disease resulting from a pituitary tumor.

Compounds of general formula (I) may be for use in preventing and/or treating a pituitary tumor inducing a Cushing's disease.

Patients suffering from a Cushing syndrome or from a Cushing's disease may suffer from central obesity, skin atrophy, muscle wasting, cardiovascular disease, diabetes, metabolic syndrome, osteoporosis, disturbed mood and/or impaired reproductive function. These morbidities are all related to an excess of corticoid hormones and define the Cushing's syndrome.

Compounds of general formula (I) may be for use in preventing and/or treating at least one morbidity associated with a Cushing's disease or a Cushing's syndrome. A morbidity associated with a Cushing's disease may be one of central obesity, skin atrophy, muscle wasting, cardiovascular disease, diabetes, metabolic syndrome, osteoporosis, disturbed mood and impaired reproductive function.

In some embodiments, the compound may be used in combination with an EGFR inhibitor. An EGFR inhibitor may be gefitinib, erlotinib, osimertinib, neratinib, cetuximab, panitumumab, dacomitinib, lapatinib, necitumumab, mobocertinib, or vandetanib, or combinations hereof. In some embodiments, an EGFR inhibitor may be gefitinib.

In some embodiments, compounds of formula (I) for use for treating a cancer, may be administered simultaneously, separately or sequentially with an EGFR inhibitor.

According to another object, the present disclosure relates to a method of treating an ACTH-dependent Cushing's syndrome comprising the administration of a therapeutically effective amount of a compound of formula (I) to a patient in need thereof.

According to another object, the present disclosure relates to a compound of formula (I) for use in the manufacture of a medicament for treating an ACTH-dependent Cushing's syndrome in a patient in need thereof.

The compounds of the disclosure may be administered to a patient in need thereof via any of the accepted modes of administration in the field. These methods may include oral, parenteral, IV, IM, subcutaneous and other systemic, aerosol, and topical forms, as well as sustained release systems like transdermal patches. An administration may be by oral route.

Compounds of the disclosure may be formulated in suitable pharmaceutical composition. A pharmaceutical composition may comprise one compound of the disclosure with at least one pharmaceutically acceptable excipient.

A pharmaceutical composition may be prepared under the form of tablets, pills, capsules, powders, aerosols, suppositories, skin patches, parenteral, and oral liquids including oil aqueous suspensions, solutions and emulsions.

When a pharmaceutical composition is in a solid form, it may include a solid pharmaceutical carrier. A solid carrier can be one or more substances, which may also act as diluents, flavouring agents, solubilizers, lubricants, suspending agents, binders, or tablet disintegrating agents; it can also be an encapsulating material. A solid carrier may be starch, sugar, talc, mannitol, povidone, magnesium stearate, and the like. A solid form such as a tablet may include a lubricant. Suitable lubricants like magnesium stearate, binders such as gelatin, and disintegrating agents like sodium carbonate in combination with citric acid may be used to form the tablets.

Tablets, powders, cachets, and capsules can be used as solid dosage forms suitable for oral administration.

Liquid form compositions include solutions, suspensions, and emulsions. For example, sterile water or propylene glycol solutions of the active compounds may be liquid preparations suitable for parenteral administration. Liquid compositions can also be formulated in solution in aqueous polyethylene glycol solution.

Aqueous solutions for oral administration can be prepared by dissolving the active component in water and adding suitable colorants, flavouring agents, stabilizers, and thickening agents as desired. Aqueous solutions for oral use can be made by dispersing the finely divided active component in water together with a viscous material such as natural synthetic gums, resins, methyl cellulose, sodium carboxymethyl cellulose, and other suspending agents known to the pharmaceutical formulation art.

Depending on the mode of administration, the pharmaceutical composition will according to one embodiment of the present invention include 0.05% to 99% weight (percent by weight), according to an alternative embodiment from 0.10 to 50% weight, of the compound of the present invention, all percentages by weight being based on total composition. A therapeutically effective amount for the practice of the present invention may be determined, by the use of known criteria including the age, weight and response of the individual patient, and interpreted within the context of the disease which is being treated or which is being prevented, by one of ordinary skills in the art.

### Methods of screening

The present disclosure relates to a use of a cysteine-containing buffer for identifying a Ubiquitin Specific Protease (USP) inhibitor. A cysteine-containing buffer of the disclosure may be used in a method for identifying or screening a USP inhibitor.

The use and the method may be for identifying USP2 or USP8 inhibitors. The use and the method may be for identifying USP8 inhibitors.

The present disclosure relates to a method for identifying a USP inhibitor, the method using at least:
- a catalytic domain of USP having a USP activity,
- a cysteine-containing buffer, and a
- substrate of said USP, said substrate being a reporter substrate or a substrate capable of being converted to a reporter molecule,
and
the method comprising at least the steps of:
a) contacting said catalytic domain of USP with a presumed USP inhibitor in said cysteine-containing buffer and said substrate in conditions suitable for the presumed USP inhibitor to bind to the catalytic domain of USP,
b) measuring an amount of said reporter substrate or of said reporter molecule obtained at step a), and
c) comparing the amount measured at step b) with a value of reference, the value of reference being obtained by contacting said catalytic domain with said substrate in absence of the presumed USP inhibitor,
wherein a deviation between the amount measured at step b) and the value of reference is indicative of an inhibition of the USP activity by said presumed USP inhibitor.

The use of a cysteine instead of dithiothreitol as in the reaction buffer for selecting USP inhibitor, in particular USP8 inhibitor, allows advantageously to identify selective and reversible inhibitor of the USP.

The USP inhibitor may be a USP8 or a USP2 inhibitor. In some embodiments, the USP inhibitor may be a USP8 inhibitor

Methods of identifying compounds suitable for inhibiting UPS, such as USP8, are carried out in conditions suitable for ensuring linearity of enzyme activity throughout the assay incubation time.

The methods may be carried in presence of a molar excess of compounds presumed to inhibit the USP.

A value of reference may be as above indicated.

A value of reference, or control, may be obtained by using, in the buffer reaction, a buffer in place of the enzyme or a diluent in place of the presumed inhibitor, such as DMSO.

In some embodiment, a value of reference is obtained by using, in the enzymatic reaction, the diluent used for the compound, such as DMSO, in place of the compound presumed to inhibit USP.

In some embodiment, a value of reference is obtained by using, in the enzymatic reaction, a buffer in place of the enzyme. That may correspond to a total (100%) inhibition of the enzyme.

The activity of USP may be proportional to a signal of a reporter substrate, for example an emitted fluorescence. An emitted fluorescent may be expressed in relative fluorescence units generated per minute (ΔRFU/min) or in relative fluorescence units at a final time point.

### Buffers

Buffers suitable for a method of the disclosure may be a buffer comprising at least a cysteine. Buffers of the disclosure are aqueous buffer.

The cysteine is present in a buffer of the disclosure as a redox compound.

A buffer of the disclosure contains cysteine as the sole redox compound.

The cysteine may be present in a buffer of the disclosure in an amount ranging from about 2 mM to about 10 mM, from about 2.5 mM to about 8 mM, from about 3.5 mM to about 6.5 mM, or may be about 5.5 mM.

The cysteine may be present in a buffer of the disclosure in an amount of about 5.5 mM.

The cysteine may be a L- or D-cysteine.

A buffer suitable for a method of the disclosure does not contain dithiothreitol (DTT).

A suitable buffer may be a phosphate buffer, a MES (morpholino-ethanesulfonic acid) buffer, a BIS-TRIS buffer, a citrate buffer, a TRIS-HCl buffer, a bicarbonate buffer, or a borate buffer. A phosphate buffer may comprise sodium phosphate monobasic and sodium phosphate dibasic.

In some embodiment, a suitable buffer may a Tris-HCl buffer.

In some embodiments, the cysteine containing buffer may comprise at least one of ovalbumin, bovine serum albumin, or human serum albumin.

A buffer may further comprise a chelating agent, such as EDTA, a surfactant, such as Tween^{®} 20 or 80, sodium acetate, HEPES, or NaCl.

A buffer suitable for the methods of the disclosure may be a Tris HCl buffer comprising Tris HCl, ovalbumin, and cysteine, such as L-cysteine.

A buffer suitable for the methods of the disclosure may be a Tris HCl buffer comprising 40 mM Tris HCl pH 7,4, 0,5mg/ml ovalbumin, and L-cysteine HCl 5,5mM.

### Reporter substrate and reporter molecule

A substrate suitable for the methods of the disclosure may be any substrate susceptible to be hydrolyzed by USP, such as USP8, and for which the remaining amount may be measured - reporter substrate - or for which the cumulating amount of the resulting metabolite may be measured - reporter molecule.

A reporter substrate or a reporter molecule are compounds for which the amounts can be measured and can be correlated with the enzyme activity. The amount of a reporter substrate or of a reporter molecule may be measured by any known techniques in the art. The reporter molecule or the reporter substrate may be separated from the reaction mixture and then dosed, for example by SDS-PAGE and measure of the intensity of the corresponding band or by ELISA. Alternatively, the reporter molecule or the reporter substrate may emit a spectrometric signal, such as a fluorescent, a luminescent, or a colorimetric signal, which can be measured which can be measured in the reaction mixture, and which can be correlated to the amount of the reporter substrate or reporter molecule.

For example, a suitable substrate may be a reporter substrate emitting a fluorescent signal which can be quenched following to the hydrolysis of the substrate by the enzyme. The measure of the fluorescent signal as a function of time or at an endpoint of time is indicative of the remaining amount of the reporter substrate and of the enzyme activity.

A suitable substrate may be a substrate susceptible to be converted into a reporter molecule emitting a fluorescent signal which can appear further to the hydrolysis of the substrate. The measure of the fluorescent signal as a function of time or at an endpoint of time is indicative of the cumulating amount of the reporter molecule and of the enzyme activity.

A suitable substrate may be a ubiquitin molecule conjugated to a leaving fluorescent leaving group. The hydrolyze of the substrate by USP results in the leave of the fluorescent leaving group and the apparition of the fluorescent signal. A suitable fluorescent group may be fluorescein and its derivatives, rhodamine and its derivatives, isothiocyanate, phycoerythrin, phycocyanin, allophycocyanin, o-phthaladehyde, fluorescamine, dansyl, umbelliferone, luciferin, an aromatic acridinium ester label, an imidazole label, an acridimium salt label, an aequorin label, 2,3-dihydrophthalazinediones, Texas Red, dansyl, Lissamine, or commercially available fluorophores such SPECTRUM ORANGE^{®} and SPECTRUM GREEN^{®} and/or derivatives of any one or more of the above. Fluorescence can be quantified using a fluorimeter.

A suitable substrate may be Ubiquitin-Rhodamine 110 (Ub-Rho) (commercially available under the ref: 60-0117-050, from Ubiquigent) or ubiquitin-7-amido-4-methylcoumarin (commercially available from Ubiquigent under the ref. 60-0116-050).

The activity of USP may be correlated to the intensity signal of a reporter substrate or of a reporter molecule, for example an emitted fluorescence. An emitted fluorescent may be expressed in relative fluorescence units generated per minute (ΔRFU/min) or in relative fluorescence units at a final time point.

In some embodiments, the USP is USP8 or USP2. In some embodiments, the USP is USP8.

### USP

The methods of the disclosure may implement any suitable ubiquitin specific protease (USP).

In some embodiments, the USP is a human USP.

The enzyme may be used a full length USP (USPFL). In case of USP8, such enzyme is commercially available, for example from BostonBiochem, under the reference E-520.

Alternatively, a purified catalytic domain of USP (USP-CD) may be used.

USPs have quite-well conserved catalytic core (or catalytic domain). The USP catalytic core can be divided into six conserved boxes that are present in all USP domains. Box 1 contains the catalytic Cys residue, box 5 contains the catalytic His, and box 6 contains the catalytic Asp/Asn residue. All boxes show several additional conserved features and residues. Boxes 3 and 4 contain a Cys-X-X-Cys motif each, which have been shown to constitute a functional zinc-binding motif. Potentially, zinc-binding facilitates folding of the USP core, helping the interaction of sequence motifs some few hundred residues apart. USP domains share a common conserved fold (https://www.ebi.ac.uk/interpro/entry/InterPro/IPR018200/).

In some embodiments, the USP is USP8 or USP2. In some embodiments, the USP is USP8. The USP8 is a human USP8.

The gene sequence and the coding sequence are available from NCBI database under accession number: NM_005154.

The full-length USP8, or its catalytic domain, can be produced by any suitable method, including recombinant and non-recombinant methods (e.g., chemical synthesis).

The protein sequence of USP8 can be available from UniProt database under accession number: P40818. The USP catalytic domain extends between amino acid positions 777-1109 of the full length sequence presented under accession number: P40818.

Recombinant techniques can involve any suitable construct and any suitable host cell, which can be a prokaryotic or eukaryotic cell, usually a bacterial or yeast host cell. Methods for introduction of genetic material into host cells include, for example, transformation, electroporation, conjugation, calcium phosphate methods and the like.

A nucleic acid sequence encoding a full-length USP, e.g., USP8, or its catalytic domain, may be present in an expression vector. The nucleic acid sequence is operably linked to a transcriptional control element, e.g., a promoter. The promoter is in some cases constitutive. The promoter is in some cases inducible. In some cases, the promoter is suitable for use (e.g., active in) a prokaryotic host cell. In some cases, the promoter is suitable for use (e.g., active in) a eukaryotic host cell.

Suitable expression vector can include various elements, including for example, promoters, selectable genetic markers (e.g., genes conferring resistance to antibiotics (for instance kanamycin, erythromycin, chloramphenicol, or gentamycin)), origin of replication (to promote replication in a host cell, e.g., a bacterial host cell), and the like.

Isolation and purification of non-naturally occurring mutated arylsulfotransferases can be accomplished according to methods known in the art. For example, a full-length USP, e.g., USP8, or its catalytic domain, can be isolated from a lysate of cells genetically modified to express the protein, for example by immunoaffinity purification. Isolated proteins can be further purified by dialysis and other methods normally employed in protein purification methods. In one example, the protein can be isolated using chromatography methods.

A full-length USP, e.g., USP8, or its catalytic domain, can be prepared in substantially pure or substantially isolated form. Purified full-length USP, e.g., USP8, or its catalytic domain, can be provided such that the polypeptide is present in a composition that is substantially free of other expressed polypeptides, e.g., less than 90%, usually less than 60% and more usually less than 50% of the composition is made up of other expressed polypeptides.

The following examples illustrate the embodiments of the invention that are presently best known. However, it is to be understood that the following are only exemplary or illustrative of the application of the principles of the present invention. Numerous modifications and alternative compositions, methods, and systems may be devised by those skilled in the art without departing from the spirit and scope of the present invention. Thus, while the present invention has been described above with particularity, the following examples provide further detail in connection with what are presently deemed to be the most practical and preferred embodiments of the invention.

### [EXAMPLES]

### Example 1: Method for identifying USP8 enzyme activity inhibitor

### Materials & Methods

To counter select oxidizing molecules, new buffering conditions were designed without dithiothreitol (DTT), but rather L-Cysteine, in order to prevent or strongly diminish the redox cycle.

In this setup, an in-house purified catalytic domain of USP8 (USP8CD) was used at a concentration of 80 nM in the presence of the artificial substrate Ubiquitin-Rhodamine 110 (Ub-Rho) (ref: 60-0117-050 Ubiquigent) at a final concentration of 0,1 µM, in conditions ensuring linearity of enzyme activity throughout the assay incubation time. Enzymatic assay was performed in a Tris HCl buffer (40 mM Tris HCl pH 7,4, Ovalbumine 0,5mg/ml, Cysteine HCl 5,5mM). Compounds to be tested were added to the reaction mix and incubated with enzyme for 20 min, before the substrate. The enzymatic reaction kinetics were monitored by measuring fluorescence elicited by the hydrolysis of the peptidyl Ub-Rho over 30 minutes. The negative control was the solvent DMSO at 1%.

PCR6236 was kindly provided by our collaborators Pr. P. Vanelle and Dr. V. Remusat from Aix-Marseille University (Fauvarque et al., 2016, EP 3 281 940 A1). Ub-Rho was purchased from Ubiquigent (ref. 60-0117-050).

### Results

Using these conditions, it was observed that the oxidizing compound PCR6236 - which is a very potent irreversible inhibitor of USP8 in the presence of DTT (Fauvarque et al., 2016) - no longer displays inhibitory activity against USP8CD (**Fig.1**).

This experimental condition thus permits to select compounds inhibiting ubiquitin specific proteases acting through a different mechanism of action than by irreversible oxidation of the catalytic cysteine residue.

### Example 2: Identification of USP8 enzyme activity inhibitors

### Materials & Methods

### Tested compounds

A set of halogenated salicylanilides were tested to define their ability to inhibit of USP8 catalytic activity and ACTH secretion by AtT-20 cells.

Further, the program F-trees similarities search on the Mcule platform (https://doc.mcule.com/doku.php?id=ftrees), a tool for scaffold hopping (i.e. identify new molecular scaffolds while maintaining activity) was used to identify analogs to on active halogenated salicylanilide, closantel. The reduced graph representation that preserves the pharmacophore characteristics of the ligands in a fuzzy way enables the identification of novel scaffolds. Six additional analogs to closantel presenting similar backbone (score >0,85) were identified. The search was done using Mcule (https://mcule.com/) "1-CLICK SCAFFOLD HOP" tool, with closantel as input molecule and the Mcule "10k diverse subset of Purchasable compounds" proprietary library as the chemical space.

Tested compounds are presented in the table below. Catalog numbers beginning with HY- were purchased from MedChemExpress, References ID beginning with Amb were purchased from Ambinter. Reference beginning with a "Z" were purchased from Molport (www.molport.com) (Supplier Enamine Ltd.).

**TABLE 1: Tested compounds**

| **ZINC number Name Cat.No./ Ref.** | **Formula** | **MW (g/mol)** | **Structure** |
|---|---|---|---|
| **ZINC2057 Salicylanilide MCE HY-B1408** | C13H11NO2 | 213 | 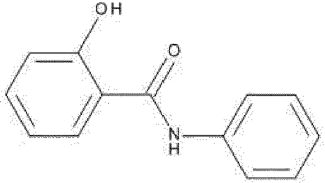 |
| **ZINC4212651 Closantel MCE HY-17596** | C22H14Cl2I2N2O2 | 663 | 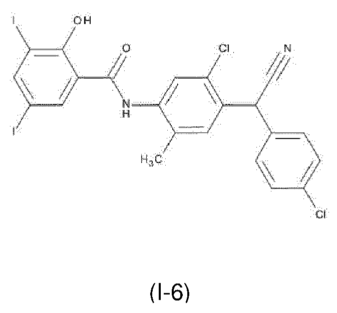 |
| **ZINC3874496 Niclosamide MCE HY-B0497** | C13H8Cl2N2O4 | 327 | 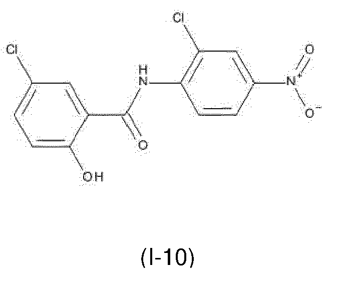 |
| **ZINC2038733 Oxyclozanide MCE HY-17594** | C13H6Cl5NO3 | 401 | 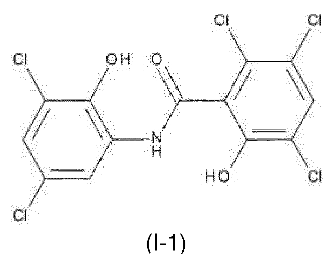 |
| | | | |
| **ZINC4181896 Rafoxanide MCE HY-17598** | C19H11Cl2I2NO3 | 626 | 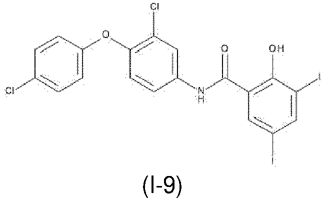 |
| **ZINC4014108 Amb8477380** | C14H11I2NO2 | 479 | 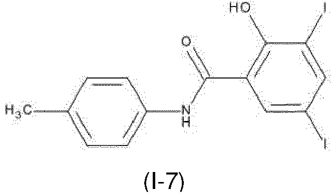 |
| **ZINC72119086 Amb4103735** | C21H14ClI2NO3 | 617 | 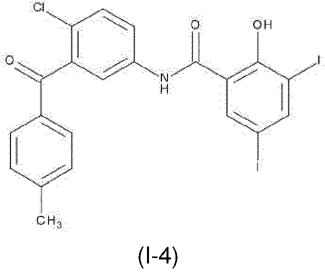 |
| **ZINC4215387 Clioxanide Amb4466501** | C15H10ClI2NO3 | 541 | 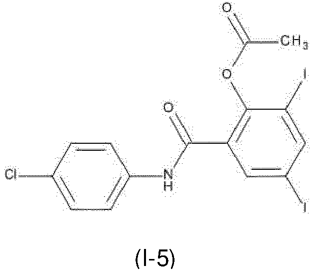 |
| **ZINC4181894 Amb8487151** | C13H7Cl2I2NO2 | 533 | |
| | | | 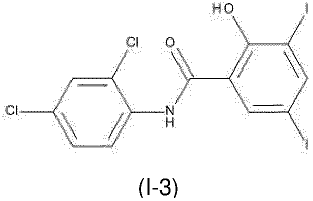 |
| **ZINC4014105 Amb8477378** | C13H7Cl2I2NO2 | 533 | 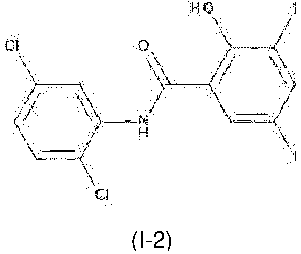 |
| **ZINC4181896 Rafoxanide Amb2704313** | C19H11Cl2I2NO3 | 626 | 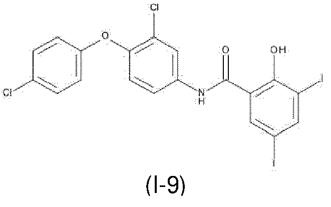 |
| **ZINC4014104 Amb1935028** | C13H8ClI2NO2 | 499 | 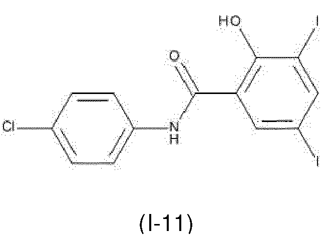 |
| **ZINC4014253 Amb8477462** | C13H7Cl2I2NO2 | 533 | |
| | | | 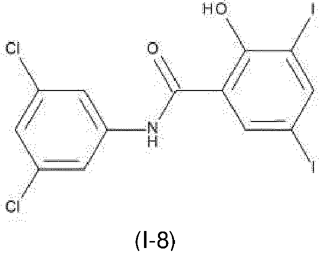 |
| **ZINC40111866 MolPort Z424433412** | C18H158rN2O2S | 403 | 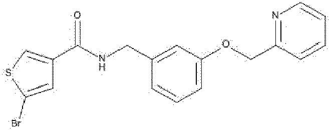 |
| **ZINC32922917 MolPort Z393438700** | C20H17FN2O2S | 368 | 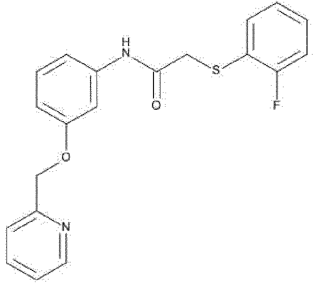 |
| **ZINC9579366 MolPort Z220328740** | C19H12ClFN2O3 | 370 | 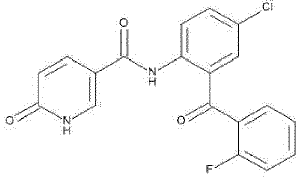 |
| **ZINC55784788 MolPort Z738998144** | C21H20N2O3 | 348 | |
| | | | 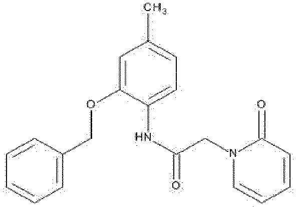 |
| **ZINC194061705 MolPort Z69118571** | C13H10INO3 | 355 | 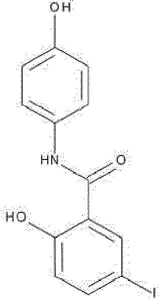 |
| **ZINC3159902 MolPort OSSL-122817** | C14H12ClNO2 | 261 | 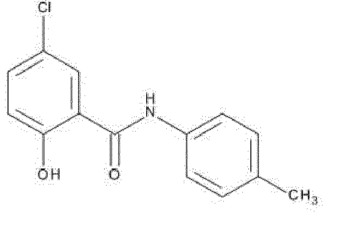 |

### USP8 inhibition

Full length USP8 (USP8FL) (BostonBiochem ref.E-520) were used at a concentration of 75 nM in the presence of a ubiquitin substrate, i.e., the Ub-Rhodamine 110 (Ub-Rho) (Ubiquigent ref. 60-0117-050) at a final concentration of 0.1 µM. Enzymatic assays were performed as described for USP8-CD except that USP-FL was used at a concentration of 75 nM. The compounds were used at a final concentration of 0.1, 1, 5, 10, 50 and 100 µM in DMSO 0.5%. The USP8 catalytic activity was monitored both before adding the substrate (T0) and after twenty minutes of incubation at room temperature (Final Time TF). The enzymatic reaction kinetics were monitored by measuring fluorescence elicited by the hydrolysis of the Ub-Rho bound over 30 minutes. The bioactive control, mimicking the desired inhibitory activity, was the reaction mix without USP8FL (mimicking 100% inhibition).

### Selectivity assays against other cysteine proteases

Selectivity towards deubiquitinating enzymes (DUBs) was assessed by testing the inhibitory efficacy of closantel over a non-DUB cysteine protease (papain), while selectivity towards USP8 compared to other DUBs was assessed by monitoring the IC₅₀ of closantel towards USP8 (Full length (also indicated FL) or USP8 catalytic domain (CD)) versus the closely similar catalytic domain of USP2 enzyme (USP2 CD) or the more distant UCH-L3 enzyme (Full length).

### Papain inhibition

Papain (Sigma ref. P4762) and casein (Merck ref.VM874644) were dissolved in 40mM TrisHCl buffers pH6.8 and pH9.5 respectively. Casein at the concentration of 0.5µg/µl was incubated with the inhibitory compounds at efficient inhibitory concentration or with DMSO for 15 min and then digested by Papain (2.5ng/µl) at RT over 45 min. The digestion samples were collected at time: 5, 15, 30 and 45min. To inactivate the protease Laemmli 2x was added and each sample was heated at 95°C for 5 min then analyzed with SDS-PAGE and stained with Coomassie Blue.

### UCH-L3 inhibition assay

UCH-L3 (BostonBiochem ref. E-325) was used at a concentration of 3nM in the presence of the Ub-Rhodamine. Enzymatic inhibition was monitored as for USP8 or USP2 CD.

### USP2 CD inhibition

The purified catalytic domain of USP2 (USP2CD, in-house purified) was used at a concentration of 16nM in the presence of the artificial substrate Ub-Rho. Inhibition assays were performed as described above.

### Reversibility (dilution) assay.

DUB activity relies on a conserved catalytic cysteine residue which is particularly sensitive to irreversible oxidation by reactive oxygen species (ROS) (Lee et al., 2013). Closantel was tested for the reversibility of the USP8 inhibition by a gel filtration assay. In this setup, purified USP8CD was used at a concentration of 60 nM and incubated for 20 min with either the inhibitory compound or with DMSO in buffered conditions. The catalytic activity of each mix was monitored on Ub-Rho substrate (Ubiquigent ref. 60-0117-050) used at a final concentration of 0.1 µM before and after filtration on a gel column (Millipore UFC30GV25). If a catalytic activity is detected after column filtration, the compound is a reversible inhibitor.

### Inhibition of ACTH secretion in pituitary cells

Mouse AtT-20 pituitary cell line were used as a powerful and well-characterized model to monitor ACTH secretion using an enzyme immunoassay (EIA) kit.

Mouse AtT-20 cells were obtained from the American Type Culture Collection and maintained on DMEM containing 10% fetal bovine serum, 100 µg/mL streptomycin and 100 U/mL penicillin at 37°C with 5% CO₂ after recovery from long-term storage on liquid nitrogen-vapor phase.

The ACTH secretion levels of AtT-20 cell culture was monitored on the supernatant of AtT-20 cells treated or not by increasing doses of compounds belonging to the family of halogenated salicylanilides chemicals.

1.25 × 10⁴ AtT20 cells were seeded on polylysine-coated 96-well culture vessels on 150µL of complete medium. After 24 hours, cells were treated with stock compounds to a final DMSO concentration of 0,5%. After 24 hours of treatment, cell culture supernatant was collected, cleared for 10 min at 1000 × g at 4°C, diluted or not in assay buffer (Phoenix Peptide ACTH EIA EK-001-21) and frozen at -80°C for subsequent processing according to the instructions of the kit's manufacturer.

### Cytotoxicity towards AtT-20 cells

The AtT-20 cells viability was monitored in the conditions used to follow ACTH secretion. The viability of treated cells was concomitantly evaluated using a Cell Viability Reagent (monitoring of the cells' reducing capacity) (PrestoBlue^{®} assay, Molecular Probes) with the aim to determine non-toxic doses of cell treatment.

After the collection of the supernatant sample for the ACTH assay in the previous step, cells were supplemented with the PrestoBlue^{™} cell viability reagent (Invitrogen^{™} A13261) at 10% final volume, and re-incubated at 37°C for 30 minutes. Fluorescence was then read with a FLUOstar OPTIMA microplate reader.

### Monitoring of POMC encoding gene expression

ACTH production depends on the activation of the proopiomelanocortin (POMC) gene encoding the ACTH precursor. In order to determine if the ACTH reduction that was observed in AtT-20 cells treated by closantel could be attributable to a reduction of POMC gene expression, POMC gene activation was monitored following cell treatment by closantel.

POMC gene mRNA transcripts were monitored by quantitative RT-PCR performed with specific primers on total RNA samples extracted from cells treated or not with increased doses of closantel (see additional methods).

3.75 × 10⁵ AtT20 cells were seeded on polylysine-coated 6-well culture microplates on 2,4 mL complete medium. After 24 hours, cells were treated with stock compounds to a final DMSO concentration of 0,5%. After 24 hours of treatment, cells were briefly rinsed with 37°C DPBS (no calcium, no magnesium modification), lysed with the LBP reagent from the NucleoSpin^{®} RNA Plus kit (Macherey-Nagel 740984.50) and then frozen at -70°C before continuing with the RNA extraction protocol as per manufacturer's instructions. 1µg RNA was used to synthesize cDNA using the AffinityScript QPCR cDNA Synthesis Kit (Agilent #600559). qPCR reaction was performed using the primers against the mouse POMC gene (5'-CCT CCT GCT TCA GAC CTC CAT AGA T-3' and 5'-GTC TCC AGA GAG GTC GAG TTT). The expression of the HPRT gene was used as a normalizer (primer pair 5'-ATG GAC AGG ACT GAA AGA CTT GCT-3' and 5'-TTG AGC ACA CAG AGG GCC ACA ATG-3'). Primers were synthesized by Eurofins Genomics. Detection was done using the Brilliant II SYBR^{®} Green QPCR Master Mix (Agilent #600828), and readings were done using a Stratagene Mx3005P equipment.

### Statistical analysis

Data was processed with the Graphpad Prism 6 (or Graphpad Prism 9) software. For both ACTH production and viability assays, data normalization was determined by QQ plot inspection. For the ACTH production assay, treatment conditions were compared using a Welch's ANOVA test followed by a Dunnett's T3 multiple comparisons test. Data for the viability assay was treated via a Kruskal-Wallis test followed by a Dunn's multiple comparisons test. For the POMC expression assay, the Ct valued of the technical triplicates were first validated via one-sample t-tests for significance using Prism. Normalization to the reference gene and the relative expression levels were then calculated with the Stratagene MxPro v4.0.

### Results

### USP8 inhibition

The selected set of halogenated salicylanilide-type compounds was able to induce more than 30% inhibition of USP8FL enzymatic activity in a dose-dependent manner (**Fig. 2A** **&** **B**).

### Selectivity assays against other cysteine proteases

Closantel was not active against papain or UCH-L3. Closantel was active both against USP8 (CD and FL) and against USP2CD.

### Reversibility (dilution) assay.

Closantel was found to inhibit USP8 in a reversible manner consistent with the screening procedure with buffering conditions favoring the selection of non-oxidizing compounds.

### Inhibition of ACTH secretion in pituitary cells

Closantel and niclosamide were shown to exhibit a significant dose-dependent inhibition of ACTH (**Fig. 3**).

Closantel and niclosamide, as well as oxyclozanide, clioxanide (Amb4466501), rafoxanide, Amb8477380, Amb4103735, Amb8487151, Amb8477378, Amb1935028, and Amb8477462 were shown to exhibit a significant inhibition of ACTH at 25 µM (**Fig. 4**).

Salicylanilide was ineffective (**Fig. 4**), as well as the closantel analogs: ZINC40111866: (C18H15BrN2O2S), cat. No. Z424433412, ZINC32922917: (C20H17FN2O2S), cat. No Z393438700, ZINC9579366: (C19H12ClFN2O3), cat. No Z220328740, ZINC55784788: (C21H20N2O3), cat. No Z738998144, ZINC194061705: (C13H10INO3), cat. No Z69118571, and ZINC3159902: (C14H12CINO2), cat. No OSSL-122817. None of the six additional compounds inhibited USP8 catalytic activity at 40 µM and none of them inhibited ACTH secretion by AT-t20 cells at 25 µM (**Fig. 5**). The compound Z424433412 presented a poorly significant (p<0,05) inhibition of ACTH secretion of 20%, a value which is however below the threshold of 30% inhibition chosen for this study; this compound was therefore considered as inactive.

### Cytotoxicity towards AtT-20 cells

The results suggest that rafoxanide and Amb1935028 may be partially cytotoxic in the experimental conditions used to monitor ACTH secretion (i.e;, 25 µM for 24hours). This cytotoxicity may account for the observed ACTH secretion inhibition by AT-t20 cells.

All other compounds displayed no toxicity when applied on AT-t20 cells at 25 µM for 24 hours **(****Fig.6****).**

Taken together, our results indicate that USP8 inhibition by closantel analogs is correlated with decreased ACTH secretion (**Fig.4** and **Fig.6**).

Further, a dose-dependent viability assay was carried out with both closantel and niclosamide (**Fig. 7**)

The results show that both closantel and niclosamide efficiently inhibits ACTH secretion at non-toxic doses and that closantel is less cytotoxic than niclosamide in AtT-20 cells.

More precisely, at 5,62 µM, closantel treatment induced no toxicity and a 20% reduction in ACTH release and at 30 µM treatment, closantel treatment still induced no toxicity and an ACTH secretion reduction as important as 60% compared to control conditions (**Fig. 3** and **Fig. 7**). Niclosamide treatment showed higher inhibition of ACTH secretion than closantel, inducing a 27% and 41% reduction of ACTH secretion levels with no significant toxicity at 0.5 µM and 1.0 µM, respectively albeit with considerable effects on viability upon higher doses that may partially account for reduced ACTH secretion.

The results concerning the USP8 inhibition, the inhibition of ACTH secretion in pituitary cells, and the Cytotoxicity towards AtT-20 cells are summarized in the table below.

**TABLE 2: USP8FL and ACTH secretion inhibition with the tested compounds**

| **ZINC number Name Cat.No./ Ref.** | **Structure** | **USP8FL inhbition IC50 [µM]** | **ACTH secretion % of control (DMSO) at 25µM** | **AtT-20 cell viability (PrestoBlu e assay) % of control (DMSO) at 25µM Mean (SD)** |
|---|---|---|---|---|
| | | **(CI: Confidenc e Interval)** | | |
| **ZINC2057 Salicylanilide MCE HY-B1408** | 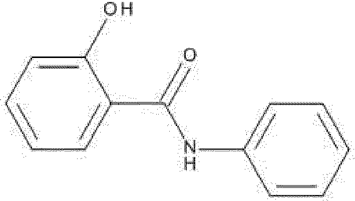 | >40µM | 99% | 105.4 (14.77) |
| **ZINC4212651 Closantel MCE HY-17596** | 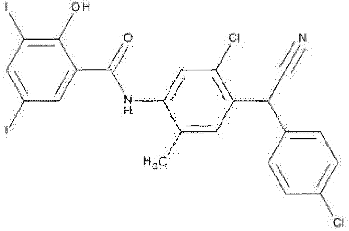 | 12µM | 65% | 104.8 (16.08) |
| | | (CI: 8.597 to 16.25) | | |
| **ZINC3874496 Niclosamide MCE HY-B0497** | 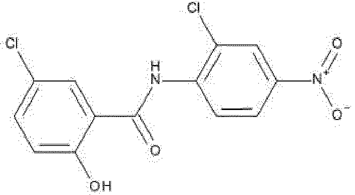 | 61 µM | 41% (at 27µM ) | 50% (12,40) (at 27µM) |
| | | (N/A) | | |
| **ZINC2038733 Oxyclozanide MCE HY-17594** | 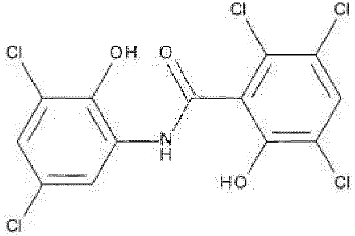 | 7.68 µM | 41% | 98.08 (14.38) |
| | | (CI: 1.164 to 14.19) | | |
| **ZINC4181896 Rafoxanide MCE HY-17598** | 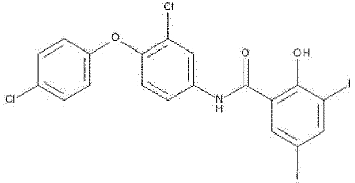 | 7.63 µM | 57% | 56.15 (3.536) |
| | | (CI: 4.399 to 10.87) | | |
| **ZINC4014108 Amb8477380** | 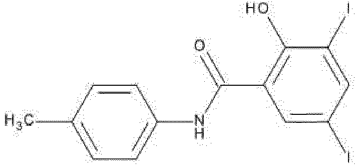 | 14.56 µM | 66% | 93.51 (11.59) |
| | | (CI: 5.914 to 49.51) | | |
| **ZINC72119086 Amb4103735** | 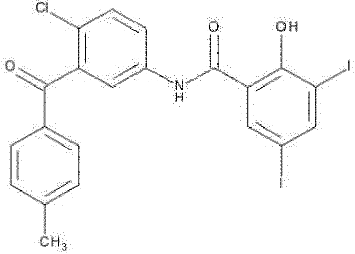 | 3.41 µM | 58% | 109.4 (18.18) |
| | | (CI : 0.9971 to 15.19) | | |
| **ZINC4215387 Clioxanide Amb4466501** | 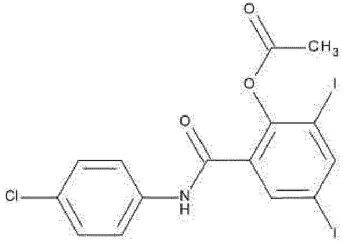 | 33.17 µM (CI : 8.710 to 363.8) | 57% | 94.21 (12.30) |
| **ZINC4181894 Amb8487151** | 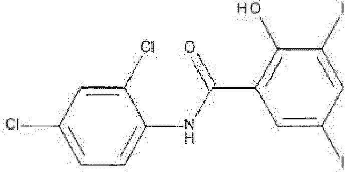 | 4.343 µM | 44% | 89.58 (10.11) |
| | | (CI : 2.339 to 8.049)) | | |
| **ZINC4014105 Amb8477378** | 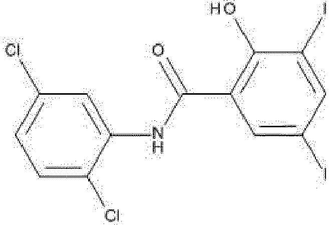 | 12.98 µM | 41% | 95.72 (11.42) |
| | | (CI : 6.758 to 27.62) | | |
| **ZINC4181896 Rafoxanide Amb2704313** | 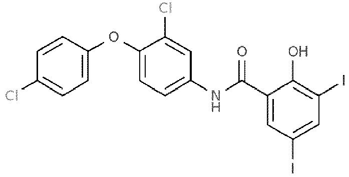 | 8.205 µM | 33% | 39.30 (6.852) |
| | | (CI: 3.817 to 18.46) | | |
| | | | | |
| **ZINC4014104 Amb1935028** | 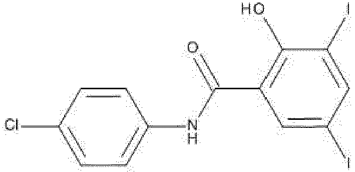 | 11,89 µM | 32% | 58.81 (34.11) |
| | | (CI: 7.298 to 20.15) | | |
| **ZINC4014253 Amb8477462** | 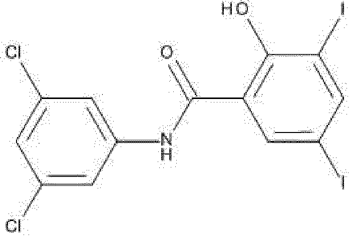 | 8.98 µM | 31% | 67.96 (17.52) |
| | | (CI: 4.782 to 17.78) | | |
| **ZINC40111866 MolPort Z424433412** | 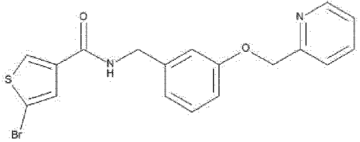 | >40µM 20/09/04 | 80% | 98.93 (14.69) |
| **ZINC32922917 MolPort Z393438700** | 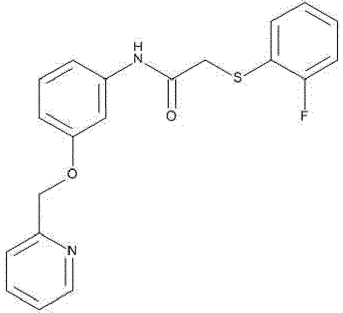 | >40µM 20/09/04 | 92% | 101.1 (16.04) |
| **ZINC9579366 MoIPort Z220328740** | 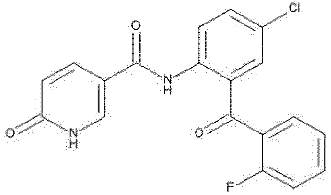 | >40µM 20/09/04 | 82% | 102.9 (15.16) |
| **ZINC55784788 MolPort Z738998144** | 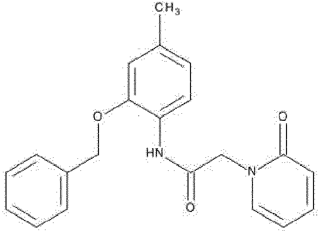 | >40µM 20/09/04 | 90% | 95.98 (15.33) |
| **ZINC194061705 MolPort Z69118571** | 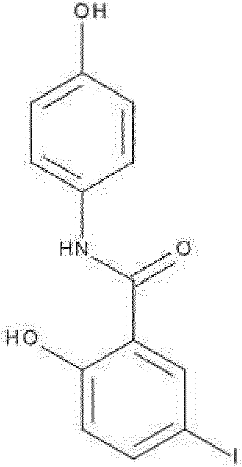 | >100µM 2022/01/26 | 101% | 106.7 (20.87) |
| **ZINC3159902 MolPort OSSL-122817** | 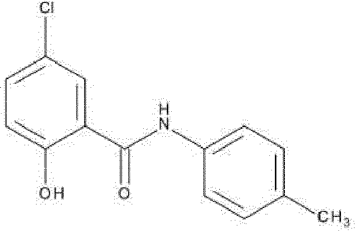 | >100µM 2022/01/26 | 118% | 75.58 (8.696) |

### Monitoring of POMC encoding gene expression

A strong decrease of POMC mRNA species was repeatedly observed (**Fig**. **8**).

This result suggests that closantel acts by negatively regulating upstream signaling pathways controlling POMC encoding gene expression. This in in accordance with the fact that USP8 constitutive catalytic activity stabilizes the EGFR, itself activating POMC gene expression through a MAPK pathway. Hence, inhibition of ACTH expression in cells treated with closantel may result from the inhibition of POMC gene expression itself resulting from EGFR stabilization provoked by USP8 inhibition.

### Conclusion

We present here the screening of a set of compounds for USP8 catalytic activity inhibition, followed by a characterization of selectivity towards other proteases and the reversibility of the inhibition. Due to the discovery of mutations of USP8 in pituitary tumors of patients of Cushing's disease and its implications for the physiopathology of the disease, we monitored the compounds capacity of decreasing ACTH release and POMC encoding gene expression (encoding the ACTH precursor) in a murine pituitary cellular model. This procedure led us to the identification of the FDA-approved molecule closantel which belongs to the class of compounds known as halogenated salicylanilides and is currently used for its anthelmintic activity in livestock. Interestingly, this compound inhibits USP8 in a reversible manner; it is active in buffering conditions using L-Cysteine that we show here prevents the selection of compounds inducing USP8 inactivation by oxidation of the catalytic Cysteine.

In addition to closantel, active analogs were identified, including niclosamide which are currently the subject of intensive drug repurposing in humans in the field of cancer treatment (Chen et al., 2018; Kadri et al., 2018) or antibacterial or antiviral drugs, and a set of nine active analogs inhibiting both USP8 and ACTH Secretion possessing at least one halogen in the position para to the hydroxyl of the salicyl ring in the salicylanilide backbone (iodine or chlorine in our case)..

### [REFERENCES]

Asari, Y., Kageyama, K., Sugiyama, A., Kogawa, H., Niioka, K., and Daimon, M. (2019). Lapatinib decreases the ACTH production and proliferation of corticotroph tumor cells. Endocr J.
Baranyai Z, Krátký M, Vosátka R, et al. In vitro biological evaluation of new anti mycobacterial salicylanilide-tuftsin conjugates. Eur J Med Chem. 2017;133:152-173. doi:10.1016/j.ejmech.2017.03.047
Ben-Shlomo, A., and Cooper, O. (2017). Role of tyrosine kinase inhibitors in the treatment of pituitary tumours: from bench to bedside. Curr Opin Endocrinol Diabetes Obes 24, 301-305.
Byun, S., Lee, S.Y., Lee, J., Jeong, C.H., Farrand, L., Lim, S., Reddy, K., Kim, J.Y., Lee, M.H., Lee, H.J., et al. (2013). USP8 is a novel target for overcoming gefitinib resistance in lung cancer. Clinical cancer research : an official journal of the American Association for Cancer Research 19, 3894-3904.
Chen CL, Lee CC, Liu FL, et al. Design, synthesis and SARs of novel salicylanilides as potent inhibitors of RANKL-induced osteoclastogenesis and bone resorption. Eur J Med Chem. 2016;117:70-84. doi:10.1016/j.ejmech.2016.04.007,
CN109851526A - The preparation method of closantel sodium
Colombo, M., Vallese, S., Peretto, I., Jacq, X., Rain, J.C., Colland, F., and Guedat, P. (2010). Synthesis and biological evaluation of 9-oxo-9H-indeno[1,2-b]pyrazine-2,3-dicarbonitrile analogues as potential inhibitors of deubiquitinating enzymes. ChemMedChem 5, 552-558.
Cristante, J., Lefournier, V., Sturm, N., Passagia, J.G., Gauchez, A.S., Tahon, F., Cantin, S., Chabre, O., and Gay, E. (2019). Why we should still treat by neurosurgery patients with Cushing's disease and a normal or inconclusive pituitary MRI. The Journal of clinical endocrinology and metabolism 104, 4101-4113.
EP 3 281 940 A1 - Heterocyclic naphthoquinones derivatives for use in the treatment of cancers including Cushing disease
Fauvarque, M.O., Mortier, M., Pillet, C., Aguilar, C., Soleilhac, E., Barette, C., Remusat, V., Terme, T., and Vanelle, P. (2016). Heterocyclic naphthoquinones derivatives for use in the treatment of cancers including Cushing disease. Patent EP3281940 Application WO2018029137A1.
Gopinath, P., Mahammed, A., Ohayon, S., Gross, Z., and Brik, A. (2016). Understanding and predicting the potency of ROS-based enzyme inhibitors, exemplified by naphthoquinones and ubiquitin specific protease-2. Chem Sci 7, 7079-7086.
Han, J., Tian, Y., Yu, L., Zhang, Q., Xu, X., Zhang, Y., Wang, J., Ma, Z., Bian, J., Luo, C., et al. (2020). Discovery of novel USP8 inhibitors via Ubiquitin-Rho-110 fluorometric assay based high throughput screening. Bioorganic chemistry 101, 103962.
Jeong, M., Lee, E.W., Seong, D., Seo, J., Kim, J.H., Grootjans, S., Kim, S.Y., Vandenabeele, P., and Song, J. (2017). USP8 suppresses death receptor-mediated apoptosis by enhancing FLIPL stability. Oncogene 36, 458-470.
Jian, F.F., Li, Y.F., Chen, Y.F., Jiang, H., Chen, X., Zheng, L.L., Zhao, Y., Wang, W.Q., Ning, G., Bian, L.G., et al. (2016). Inhibition of Ubiquitin-specific Peptidase 8 Suppresses Adrenocorticotropic Hormone Production and Tumorous Corticotroph Cell Growth in AtT20 Cells. Chin Med J (Engl) 129, 2102-2108.
Kadam, Jagannath & George, Kondolo & Dhamnaskar, Rajendra & LOKHANDE, Dr. MANOHAR. Synthesis of Closantel as Effective Reaction Media for Hydrotropes. Journal of Chemical, Biological and Physical Sciences, 2014, 44. 918-926.
Lee, J.G., Baek, K., Soetandyo, N., and Ye, Y. (2013). Reversible inactivation of deubiquitinases by reactive oxygen species in vitro and in cells. Nature communications 4, 1568.
Lefournier, V., Martinie, M., Vasdev, A., Bessou, P., Passagia, J.G., Labat-Moleur, F., Sturm, N., Bosson, J.L., Bachelot, I., and Chabre, O. (2003). Accuracy of bilateral inferior petrosal or cavernous sinuses sampling in predicting the lateralization of Cushing's disease pituitary microadenoma: influence of catheter position and anatomy of venous drainage. The Journal of clinical endocrinology and metabolism 88, 196-203.
Lonser, R.R., Nieman, L., and Oldfield, E.H. (2017). Cushing's disease: pathobiology, diagnosis, and management. J Neurosurg 126, 404-417.
Ma, Z.Y., Song, Z.J., Chen, J.H., Wang, Y.F., Li, S.Q., Zhou, L.F., Mao, Y., Li, Y.M., Hu, R.G., Zhang, Z.Y., et al. (2015). Recurrent gain-of-function USP8 mutations in Cushing's disease. Cell research 25, 306-317.
Oh, Y.M., Lee, S.B., Choi, J., Suh, H.Y., Shim, S., Song, Y.J., Kim, B., Lee, J.M., Oh, S.J., Jeong, Y., et al. (2014). USP8 modulates ubiquitination of LRIG1 for Met degradation. Scientific reports 4, 4980.
Perez-Rivas, L.G., and Reincke, M. (2016). Genetics of Cushing's disease: an update. J Endocrinol Invest 39, 29-35.
Perez-Rivas, L.G., Theodoropoulou, M., Puar, T.H., Fazel, J., Stieg, M.R., Ferrau, F., Assie, G., Gadelha, M.R., Deutschbein, T., Fragoso, M.C., et al. (2018). Somatic USP8 mutations are frequent events in corticotroph tumor progression causing Nelson's tumor. European journal of endocrinology 178, 59-65.
Pivonello, R., De Leo, M., Cozzolino, A., and Colao, A. (2015). The Treatment of Cushing's Disease. Endocr Rev 36, 385-486.
Reincke, M., Sbiera, S., Hayakawa, A., Theodoropoulou, M., Osswald, A., Beuschlein, F., Meitinger, T., Mizuno-Yamasaki, E., Kawaguchi, K., Saeki, Y., et al. (2015). Mutations in the deubiquitinase gene USP8 cause Cushing's disease. Nature genetics 47, 31-38.
Rong, Z., Zhu, Z., Cai, S., and Zhang, B. (2020). Knockdown of USP8 Inhibits the Growth of Lung Cancer Cells. Cancer management and research 12, 12415-12422.
Sarkis, P., Rabilloud, M., Lifante, J.C., Siamand, A., Jouanneau, E., Gay, E., Chaffanjon, P., Chabre, O., and Raverot, G. (2018). Bilateral adrenalectomy in Cushing's disease: Altered long-term quality of life compared to other treatment options. Ann Endocrinol (Paris).
Satyavan Sharma, Nitya Anand, Chapter 9 - Salicylanilides, Editor(s): Satyavan Sharma, Nitya Anand, Pharmacochemistry Library, Elsevier, Volume 25, 1997, Pages 239-257
Sun, J., Shen, D., Zheng, Y., Ren, H., Liu, H., Chen, X., and Gao, Y. (2020). USP8 Inhibitor Suppresses HER-2 Positive Gastric Cancer Cell Proliferation and Metastasis via the PI3K/AKT Signaling Pathway. OncoTargets and therapy 13, 9941-9952.
Tian, Y., Liu, K., Liu, R., Qiu, Z., Xu, Y., Wei, W., Xu, X., Wang, J., Ding, H., Li, Z., et al. (2022). Discovery of Potent Small-Molecule USP8 Inhibitors for the Treatment of Breast Cancer through Regulating ERalpha Expression. Journal of medicinal chemistry 65, 8914-8932.
Treppiedi, D., Di Muro, G., Marra, G., Barbieri, A.M., Mangili, F., Catalano, R., Serban, A., Ferrante, E., Locatelli, M., Lania, A.G., et al. (2021). USP8 inhibitor RA-9 reduces ACTH release and cell growth in tumor corticotrophs. Endocrine-related cancer 28, 573-582.
Uzilov, A.V., Taik, P., Cheesman, K.C., Javanmard, P., Ying, K., Roehnelt, A., Wang, H., Fink, M.Y., Lau, C.Y., Moe, A.S., et al. (2020). USP8 and TP53 drivers are associated with CNV in a corticotroph adenoma cohort enriched for aggressive tumors. The Journal of clinical endocrinology and metabolism.
Vinsova J, Imramovsky A, Buchta V, et al. Salicylanilide acetates: synthesis and antibacterial evaluation. Molecules. 2007;12(1):1-12. Published 2007 Jan 1. doi:10.3390/12010001

## Claims

1. A compound of general formula (I): 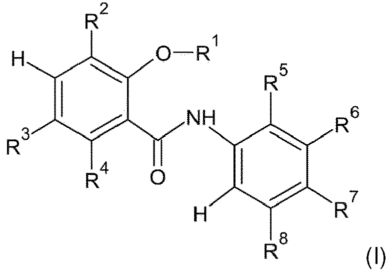
for use for treating an adrenocorticotropin hormone (ACTH)-dependent Cushing's syndrome,
wherein
- R¹ is H or a -C(O)-R⁹, wherein R⁹ is a C₁-C₃ linear or branched, saturated or unsaturated, alkyl group,
- R² is H or a halogen,
- R³ is a halogen,
- R⁴ and R⁸ are, independently, H or a halogen,
- R⁵ is H, a C₁-C₃, linear or branched, saturated or unsaturated, alkyl group, OH, or a halogen,
- R⁶ is H, a halogen, or -C(O)-Ar, wherein Ar is a C₆-C₁₀ aryl group, Ar being optionally substituted with a C₁-C₃ linear or branched, saturated or unsaturated, alkyl group,
- R⁷ is H, a halogen, a C₁-C₃, linear or branched, saturated or unsaturated, alkyl group, -NO₂, or -B-Ar, wherein B is O or -C(CN)-, and Ar is a C₆-C₁₀ aryl group, Ar being optionally substituted with a halogen,
wherein if R² is H, then at least one of R⁵, R⁶, R⁷ and R⁸ is not H,
or one of its pharmaceutically acceptable salts.

2. The compound for use according to claim 1, wherein the ACTH-dependent Cushing's syndrome is a Cushing's disease or a Cushing's syndrome associated with an ectopic ACTH secretion.

3. The compound for use according to any one of claims 1 or 2, wherein R¹ is H or - C(O)-CH₃.

4. The compound for use according to any one of claims 1 to 3, wherein R² is H, I or CI.

5. The compound for use according to any one of claims 1 to 4, wherein R⁴ is H or Cl.

6. The compound for use according to any one of claims 1 to 5, wherein R⁵ is H, OH, CH₃ or Cl

7. The compound for use according to any one of claims 1 to 6, wherein R⁶ is H, Cl, or C(O)-Phe-*p*CH₃.

8. The compound for use according to any one of claims 1 to 7, wherein R⁷ is H, Cl, CH₃, NO₂, O-Phe-pCl or C(CN)-Phe-pCl.

9. The compound for use according to any one of claims 1 to 8, wherein
- R¹ is H or a -C(O)-CH₃,
- R² and R³ are, independently, I or Cl,
- R⁴ and R⁸ are, independently, H or Cl,
- R⁵ is H, -CH₃, OH, or Cl,
- R⁶ is H, Cl, or C(O)-Phe-pMe,
- R⁷ is H, Cl, CH₃, NO₂, O-Phe-*p*Cl or -C(CN)-Phe-*p*Cl.

10. The compound for use according to any one of claims 1 to 9, wherein the compound is one of: 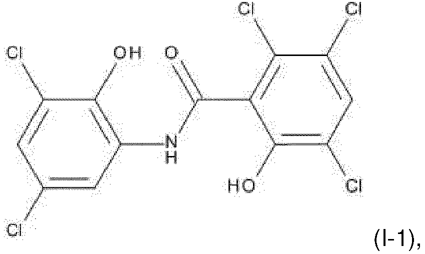 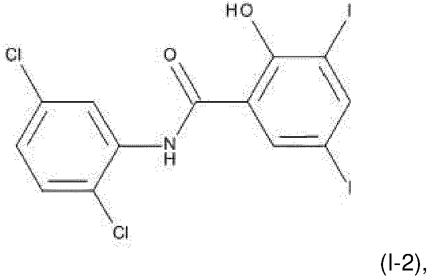 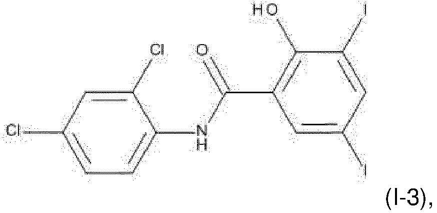 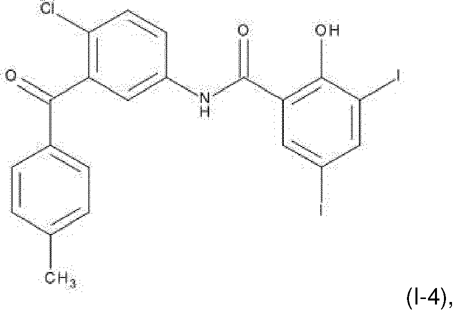 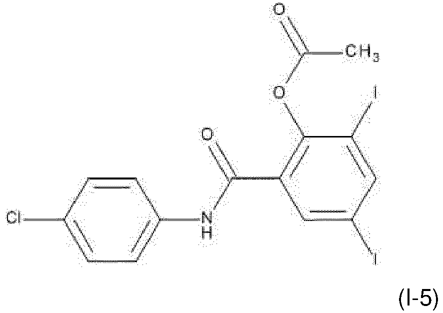 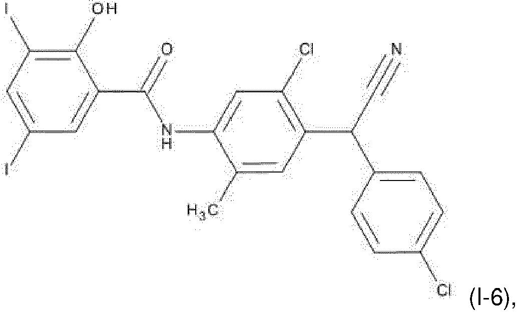 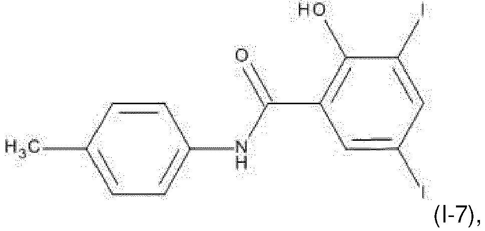 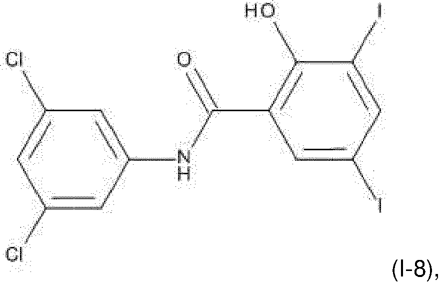 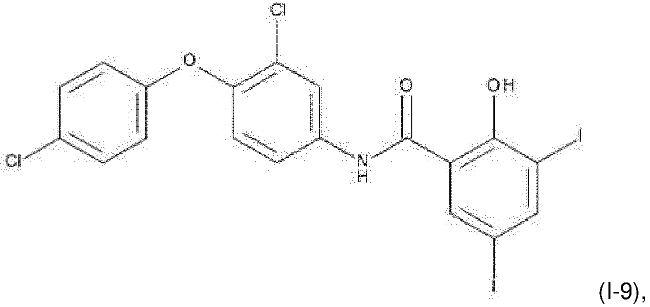 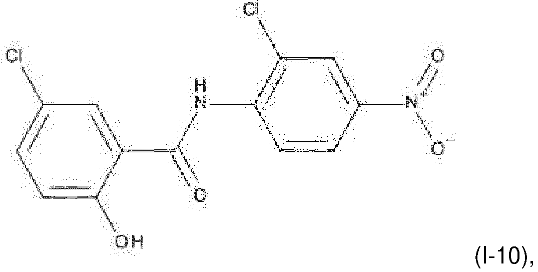 and

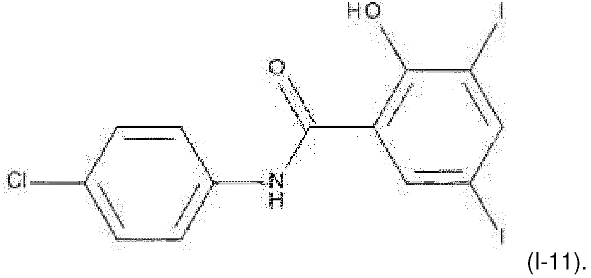

11. The compound for use according to anyone of claims 1 to 10, wherein said compound is used in combination with an EGFR inhibitor.

12. Use of a cysteine-containing buffer for identifying a Ubiquitin Specific Protease (USP) inhibitor.

13. A method for identifying a Ubiquitin Specific Protease (USP) inhibitor,
the method using at least:
- a catalytic domain of USP having a USP activity,
- a cysteine-containing buffer, and a
- substrate of said USP, said substrate being a reporter substrate or a substrate capable of being converted to a reporter molecule,
and
the method comprising at least the steps of:
a) contacting said catalytic domain of USP with a presumed USP inhibitor in said cysteine-containing buffer and said substrate in conditions suitable for the presumed USP inhibitor to bind to the catalytic domain of USP,
b) measuring an amount of said reporter substrate or of said reporter molecule obtained at step a), and
c) comparing the amount measured at step b) with a value of reference, the value of reference being obtained by contacting said catalytic domain with said substrate in absence of the presumed USP inhibitor,
wherein a deviation between the amount measured at step b) and the value of reference is indicative of an inhibition of the USP activity by said presumed USP inhibitor.

14. The use according to claim 12 or the method according to claim 13, wherein the cysteine is present in the buffer in an amount ranging from about 3 mM to about 8 mM.

15. The method according to claim 13 or 14, wherein the catalytic domain of the USP is provided as a full-length USP or as a fragment of USP containing said catalytic domain.
